# NEW EUROPEAN PATENT SPECIFICATION

(11) **EP 0 352 295 B2**
(45) Date of publication and mention of the opposition decision: **10.04.1996**
(45) Mention of the grant of the patent: 16.06.1993
(21) Application number: 88903702.4
(22) Date of filing: 30.03.1988
(51) Int. Cl.: A61K 9/16, A61K 9/50, A61K 49/00

(54) **BIODADHESION DRUG CARRIERS FOR ENDOTHELIAL AND EPITHELIAL UPTAKE AND LESIONAL LOCALIZATION OF THERAPEUTIC AND DIAGNOSTIC AGENTS**
BIOADHÄSIVER ARZNEITRÄGER ZUR ENDOTHELIALEN UND EPITHELIALEN AUFNAHME UND LÄSIONALEN LOKALISIERUNG THERAPEUTISCHER UND DIAGNOSTISCHER STOFFE
SUPPORT DE MEDICAMENTS A BIO-ADHESION POUR ABSORPTION ENDOTHELIALE ET EPITHELIALE ET LOCALISATION D'AGENTS THERAPEUTIQUES ET DE DIAGNOSTIC

(30) Priority: 01.04.1987 US 33432
(43) Date of publication of application: 31.01.1990
(73) Proprietor: Access Pharmaceuticals, Inc., Dallas, Texas 75207 (US)
(72) Inventor: RANNEY, David F., Dallas, TX 75234 (US)
(74) Representative: Dost, Wolfgang, Dr.rer.nat., Dipl.-Chem.
(86) International application number: PCT/US88/01096
(87) International publication number: WO 88/07365

(56) References cited:
- EP-A- 87 786
- EP-A- 167 825
- EP-A- 240 424
- CA-A- 1 168 150
- DE-A- 3 700 911
- GB-A- 1 516 348
- GB-A- 2 041 517
- GB-A- 2 185 397
- Biochem Pharmacology.vol. 35 p. 1063. 1986.

## Description

Until recently, the localization of intravascular drugs in body tissues has depended on chemical partitioning across microvascular barriers into the tissue compartments of multiple body organs. This resulted in only 0.01% to 0.001% of the injected dose actually reaching the intended targets. Approximately 20 years ago, drugs were entrapped in liposomes and microspheres. This modified the initial biodistributions and redirected them to phagocytes in the reticluloendothelial organs: liver, spleen and bone marrow.

In 1978, the present inventor and coworkers (Widder, et al., Proc. Am. Assn. Cancer Res., V. 19, p 17 (1978)) developed a moans to co-entrap drug plus magnetite in microspheres which could be injected intravenously and localized magnetically in the tissue compartments of nonreticuloendothelial target organs (e.g., lung and brain). Magnetic capture was accomplished by selective dragging of the particles through the vascular endothelium into normal tissues and tissue tumors positioned adjacent to an extracorporeal magnet of sufficient strength (0.5 to 0.8 Tesla) and gradient (0.1 Tesla/mm). Although this technique was highly efficient and deposited between 25% and 50% of an injected dose in the desired target tissue, it was also a very complicated approach which had the following major disadvantages: 1) restriction of use to specialized medical centers; 2) permanent disposition of magnetite in target tissue; 3) focal overdosing of drug due to inhomogeneity of the capturing magnetic field; and 4) application to a very limited number of therapeutic agents. In the process of studying magnetic targeting, however, it was learned that slow (controlled) release of toxic drugs from entrapment-type carriers (microspheres) protected the normal cells within the local tissue environment from drug toxicity and still gave effective treatment of tumor cells and microorganisms.

When monoclonal antibodies became generally available for animal and clinical research, it was hoped that antibody-drug conjugates would limit the biodistribution of toxic agents and cause them to become deposited in foci of disease (tumors and infections) which were located across the microvascular barrier within target tissues. Unfortunately, most monoclonal antibodies were (and are still) obtained from mice, making them immunologically foreign to human recipients. Conjugation of drugs at therapeutically relevant substitution ratios makes the monoclonal antibody derivatives even more foreign and impairs their binding specificities. Hence, antibody-drug conjugates are cleared rapidly by the liver, in a fashion similar to that for liposomes. Importantly, their localization in most solid tumors is even further impaired by the presence of a partially intact microvascular barrier which separates the tumor tissue (interstitium) from the bloodstream. This allows only about 1% to 7% (at best) of the injected dose to reach nonreticuloendothelial targets. Selected lymphomas and leukemias provide exceptions to this rule because of a greater natural breakdown of this vascular barrier. However, for the vast majority of solid tumors and infections, a general-purpose method is still needed to deliver drugs efficiently across microvascular barriers in a depot (controlled release) form.

Such a form of drugs is necessary in order to protect vascular endothelium and normal tissue cells from the toxic effects of drugs, protect drug from endothelial and tissue metabolism during transit, and make drug bioavailable at a controlled therapeutic rate within the target tissues and tissue lesions.

Active endothelial transport has been demonstrated for small molecules (e.g., glucose and insulin), however, no studies other those that of the present inventor have shown such transport for larger molecules or molecules carried in a cargo format. Present examples show that transendothelial migration of particles and molecular aggregates larger than ca. 2 nm in diameter are accelerated by the application of surface coatings which bind multiply to receptors or antigens which are either synthesized by endothelium or are synthesized at other sites but become tightly associated with the endothelial surface. (Ranney, Biochem. Pharmacology, V. 35, No. 7, pp. 1063-1069 (1986)).

CA-A-1 168 150 discloses a conjugate of albumin and a therapeutic agent which is made targetable by chemically linking the conjugate to an agent with binding specificity for receptors on the target cells, such as leukemia-like-tumors and hepatocytes. The albumin-carrier is included in the conjugate in an amount sufficient to mask the antigenicity of the therapeutic agent. The conjugate is delivered to the specific cell-surface receptor and internalized via the receptor itself.

The present invention involves a drug carrier or a diagnostic carrier composition comprising a drug or diagnostic agent in combination with a multivalent binding substance which binds to determinants of endothelia or epithelia that separate the blood compartment or external body surfaces from underlying tissues or sites of disease, said binding substance inducing transendothelial or transepithelial transport of the drug or diagnostic agent across said endothelia or epithelia into proximal tissues or sites of disease.

Accordingly, the present invention involves a composition of matter comprising a carrier having a surface, at least two molecules of drug or diagnostic agent contained by the carrier and a multivalent binding agent specific for endothelial surface determinants. At least a portion of said binding agent is attached to the surface of the carrier. The carrier has a size of less than 3 micrometer (µm). The binding agent is one which bioadheres to endothelial surface determinants and induces envelopment of the carrier by endothelial cells of a vascular wall and transfer across said wall to proximal tissues. The term bioadhere as used herein means interactions characteristically encountered in biological systems involving multiple molecular and usually noncovalent bonds.

The carrier involved in the method and composition of matter of the present invention preferably comprises one or more of macromolecules, microaggregates, microparticles, microspheres, nanospheres, liposomes and microemulsions. The endothelial surface determinants are those characteristic of endothelial tissues, some of which may be defined further as being enhanced in quantity when proximal to tissue lesions. These endothelial surface determinants comprise: for example, Factor VIII antigen, Interleukin I receptor, endothelial thrombomodulin, endothelial tissue factor, subendothelial tissue moieties, fibrin D-D dimer and GP 2b/3a glycoprotein complex.

The multivalent binding agent of the present invention is preferably a substance such as heparin, a heparin fragment or Ulex Europaeus I lectin. In certain cases an antibody directed toward endothelial surface antigens could be utilized as the multivalent binding agent. The multivalent binding agent of the present invention may also be directed toward subendothelial tissue moieties such as laminin, type IV collagen, fibronectin or a fibronectin fragment chemotactic for monocytes. These subendothelial moieties may, for example because of lesion formation, be exposed to vascular fluids and thus bind and/or envelop the composition of matter of the present invention. The composition of matter of the present invention may comprise a multivalent binding agent which binds to vascular endothelium via endothelial surface receptors, surface enzymes, substances which coat the endothelial surface or substances which immediately underly the endothelium and may be deposited, exposed or altered in normal vascular endothelium or proximal to foci of tissue or endothelial disease.

The composition of matter of the present invention may be used in a procedure involving binding of a sample thereof to endothelia and an induction of the endothelia to totally or partially envelop the bound sample in, for example, less than 10 to 15 minutes. The interaction of the composition of matter of the present invention with endothelia may produce an induction of the endothelia to undergo transient separation or opening, thereby exposing subendothelial determinants for which the composition of matter may also have binding affinity. The composition of matter of the present invention may, by interaction with endothelia, produce an induction of total or partial sequestration of an associated drug or diagnostic agent at an early time when it still resides in or protrudes into an associated vascular lumen.

The composition of matter of the present invention may be characterized by the interaction of a sample thereof with endothelia which produces an acceleration of transport of the sample across at least one of associated vascular endothelial and/or subendothelial structures into a proximal tissue compartment. The interaction of a sample of the composition of matter of the present invention with endothelia may result in improvement of the efficiency with which an associated drug or diagnostic agent migrates across the endothelia and associated structures such that a reduced total dose of drug or diagnostic agent may be administered to obtain effects comparable to a significantly higher dose of free drug or diagnostic agent.

The composition of matter of the present invention is preferably a microsphere in certain embodiments. Such a microsphere comprises a matrix and is less than 3 µm in diameter. The matrix is preferably a carbohydrate and may be a carbohydrate such as heparin which also has multivalent binding capabilities. Dextran is also a preferred matrix and may preferably be coated with a multivalent binding agent such as heparin, for example. In this latter case the composition of matter of the present invention is preferably about 10% (w/w) heparin.

A drug or diagnostic agent comprised in the composition of matter of the present invention may be an antifungal polyene macrolide such as amphotericin B. The amphotericin B or other hydrophobic drug or diagnostic agent may be in a cyclodextrin complex. The drug or diagnostic agent such as amphotericin B may be in a controlled-release form, for example within internally entrapped micelles of pluronic F68™ block copolymer, polyoxypropylene-polyoxyethylene.

The composition of matter of the present invention may preferably comprise a microsphere carbohydrate matrix and, as a multivalent binding agent, an exposed or covert lectin capable of binding endothelial surface determinants, enzymes, epi-endothelial or subendothelial substances.

The composition of matter of the present invention, in one preferred embodiment, comprises a carrier having a surface, at least two molecules of drug or diagnostic agent contained by the carrier, a multivalent binding agent specific for endothelial determinants, at least a portion of said binding agent being attached to the surface of said carrier and a removable coating which renders the multivalent binding agent unexposed to external contacts. The removable coating is a coating subject to removal by a triggering event. The triggering event is a condition such as lowered pH, temperature alteration, contact with normal endothelia,, contact with abnormal endothelia, altered enzyme levels or physical changes induced by application of external forces such as radiofrequency, ultrasound, magnetism or electricity.

The composition of matter of the present invention, with or without a removable coating may be one in which the multivalent binding agent is a lectin with affinity for endothelial, epi- or subendothelial determinants. In one preferred embodiment the lectin is Ulex Europaeus I lectin and the removable coating is fucose, fucosyl albumin or albumin-fucosyl amine.

The composition of matter of the present invention may comprise a multivalent binding agent which is an antibody with affinity for endothelial or subendothelial binding sites. The multivalent binding agent of the present invention also may be a substrate for an endothalial or epi-endothelial enzyme; a peptide, for example benzoyl-phenyalanyl-alanylproline, which has a substrate affinity for endothelial angiotensin converting enzyme.

In another preferred embodiment of the present invention, the drug or diagnostic agent and the multivalent binding agent are the same and comprise a molecular microaggregate of 1 to 200 nanometers in molecular diameter, most preferably where the drug or diagnostic agent and the multivalent binding agent are the same and comprise a molecular microaggregate of heparin of about 1 to 200 nanometers in molecular diameter.

The composition of matter of the present invention is, in a preferred embodiment, in a pharmaceutically acceptable solution suitable for intravascular or other parenteral injection.

Methods of use of the composition of matter of the present invention comprise administration to an animal of a carrier having a surface, at least two molecules of drug or diagnostic agent contained by the carrier and a multivalent binding agent specific for endothelial surface determinants, at least a portion of said binding agent being attached to the surface of said carrier as described above. The above composition of matter is preferably contained in a pharmaceutically acceptable carrier. The multivalent binding agents are selected for the particular targeted sites, most especially the endothelia. The drug or diagnostic agent is selected according to the particular lesion being treated or the diagnostic method being utilized. The carrier may be a natural or synthetic polymer.

Figure 1 is a lung tissue section stained with PAS, which is representative of the test mice sacrificed 2-5 minutes after intravenous injection of the unheated, acetone-stabilized heparin microspheres.

Figure 2 is a lung tissue section stained with PAS, which is representative of the test mice sacrificed 10 minutes after intravenous injection of the same heparin microspheres as in Figure 1.

Figure 3 is a lung tissue section stained with PAS, which is representative of the test mice sacrificed 2-5 minutes after intravenous injection of the fucose-blocked, Ulex Europaeus agglutinin I-coated spheres of Example 4.

Figure 4 is a lung tissue section stained with a reticulin stain, which is representative of the test mice sacrificed at 10 minutes after intravenous injection of the identical fucose-blocked, Ulex Europaeus agglutininicoated spheres of Example 3.

Figure 5 is a lung tissue section stained with PAS, which is representative of the test mice sacrificed 20 minutes after intravenous injection of the identical fucose-blocked spheres of Examples 3 and 4.

Figure 6 is a representative example of control microsphere (M_{c}) of plain agarose which is present within a lung microvessel (V) 10 minutes after intravenous injection.

The present invention involves nontoxic, biodegradable small microspheres (less than 3 micrometers (µm) in size) and microaggregates (1-200 nanometers, nm) comprising (or coated with) endothelialbinding substances. These substances induce the following serial steps upon intravenous injection of particles into test rodents: 1) endothelial bioadhesion; 2) rapid (2-minute) endothelial envelopment (partial or total) of the particles (microaggregates); 3) a facilitated (accelerated) migration of intact drug-carrier particles across microvessels into the tissue compartment; (which is largely complete within 10 to 20 minutes of injection); and 4) delayed release of drug (or diagnostic agent) from a microsphere formulation of envelopment carrier which is known to correlate with controlled bioavailability of drug within the target tissue (lesion) in vivo.

The examples presented herein include three major approaches for compositions of matter serving as formulation carriers for efficient, nonmagnetic drug localization in normal and diseased tissues, either in the presence or absence of potentially competing receptors on the surfaces of circulating red cells, white cells or platelets. These approaches are as follows: 1) microparticles (and microaggregates) comprising (and coated with) heparins which bind to the complementary heparins and heparan sulfates present on normal endothelium throughout the body (lung and brain binding are documented below); 2) microparticles with surface-conjugated Ulex Europaeus agglutinin I, a glycoprotein which binds to factor VIII antigen present on the luminal surface of endothelium and which is reported to be present at increased densities in foci of disease (Loesberg et al., Biochem. Biophys. Acta, V. 763, pp. 160 (1983)); 3) microparticles with surface-conjugated Ulex Europaeus agglutinin I, in which the factor VIII antigen-binding site is of the Ulex agglutinin blocked noncovalently by addition of the sugar hapten, L-fucose, in order to render this site covert (reversibly coated) and prevent its binding of potentially similar receptors on circulating red blood cells. Surface-coated microcarriers may also make use of interleukin 1 and its receptor sites induced by disease on the surface of vascular endothelium (Libby et al., Fed. Proc., V. 45, p. 1074 (1986)).

For these examples, initial morphometric data indicated that at least 25% of the injected carrier migrated across microvessels of the first target organ encountered, namely, lungs by the intravenous route, and brain by the carotid arterial route. Hence, these new carriers are (by a factor of five) the most efficient general-purpose drug delivery devices described. In one example, microparticles (0.1 to 0.6 µm) of amphotericincyclodextrin which released the drug at a very slow rate (t 1/2 greater than about 36 hours) were entrapped within larger (5 to 25-µm) macroparticles of a more rapidly degrading heparin matrix (t 1/2 about 15-minutes in flowing blood and blood amylase). Such a hybrid microcarrier allows for both slow release of the extravascular drug within tissues and rapid degradation of the fragments remaining within microvessels. The latter property minimizes transient disruption of microvascular blood flow which might otherwise occur upon infusion of therapeutically relevant doses of the microcarrier. This formulation comprises a true "cellular drug carrier" because it mimics the morphology and function of white blood cells (living macroparticles), which migrate into tissue lesions and release lysosomal enzymes and lymphokines (biopharmaceuticals) as a controlled rate from their intracellular granules (living microparticles).

From the results of the present invention and known biological functions and relationships involving endothelial and related binding substances, the following extensions of the present technology involving multivalent binding agents and variations thereof appear readily accomplished. These extensions may be grouped as relating to multivalent binding agents as follows:

### GROUP I. Substances which bind to native endothelium such as:

1. Heparin
2. Heparan sulfate
3. Heparin fragments and synthetic analogues which bind antithrombin III (pentasaccharides, hexasaccharides and oligosaccharides)
4. Ulex Europaeus I agglutinin (binds factor VIII antigen)
5. F-met-leu-phe
6. t-boc-leu-phe-leu-phe
7. Benzoyl-phe-ala-pro (BPAP, binds angiotensin converting enzyme)
8. Other inhibitors of angiotensin converting enzyme
9. 5'-nucleotides (bind 5'-nucleotidase)
10. Inactive congeners of the biogenic amines, 5-hydroxytryptamine and norepinephrine
11. Insulin and inactive insulin analogues
12. Transferrin
13. Prostaglandins E, F and stable congeners
14. Peptide substrates and inhibitors of tissue plasminogen activator (tPA)
15. Albumins and glycosylated albumins
16. Cationic ferritin
17. Low density lipoproteins (LDL)
18. Hirudin-inhibited thrombin (binds thrombomodulin)
19. Antibodies against (and receptor molecules for): Surface carbohydrates of:
   1. Central lymph-mode endothelium (MEL-14 and MECA-367 Ab's)
   2. Peripheral lymph-mode-endothelium (MECA-79 Ab)
   3. Panendothelium (MECA-325)
   4. Capillary-level endothelium with organ specificity (e.g.,lung, liver, and brain endothelial antibodies)
20. Negatively charged polysaccharides or oligosaccharides such as, for example:
   a. Dextran sulfate
   b. Dermatan sulfate
   c. Chondroitin sulfate, and
   d. Hyaluronic acid

### GROUP II. Substances which bind preferentially to activated and diseased endothelium

1. Ulex Europaeus I agglutinin
2. Ulex Europaeus I agglutinin, reversibly blocked with:
   a. Fucose
   b. Fucosyl albumin
   c. Albumin-fucosyl amine
   d. Other neoglycoproteins
   e. Aminated carbohydrates
3. Cytoadhesion molecules with affinity for activated endothelium:
   a. ICAM-1
   b. LFA-1
   c. Mac-1
   d. P50
   e. VLA molecules
4. Interleukin I
5. Antibodies against (and receptor molecules for):
   a. Endothelial leukocyte adhesion molecule, ELAM (H4/18 and H 18/7 Ab's)
   b. Endothelial tissue factor, tf
   c. Endothelial-associated, fibrin D-D dimer
   d. Class II histocompatibility antigens, Ia and HLA-Dr
   e. Fc receptors
   f. Mo3e surface antigens
   g. Factor VIII antigen
   h. Glycoprotein IIb
   i. Glycoprotein IIIa
   j. Glycoprotein IIb/IIIa complex
   k. II-I receptor of endothelium
   l. "Extra domain" of fibronectin, ED

### GROUP III. Substances which bind to subendothelial molecules and structures exposed by endothelial activation and disease:

1. Ricinus communis agglutinin I (binds to basement membrane molecules)
2. Antibodies against (and receptor molecules for):
   a. Fibronectin
   b. Fibronectin fragments (e.g., monocyte chemotactic fragment)
   c. Laminin
   d. Intercellular adhesion molecules (e.g., ICAM-1)
   e. Type IV collagen
   f. Basement membrane molecules (anti-GBM antibody).

An additional aspect of the present invention, is the formulation of microcarriers in which the endothelialbinding ligands are themselves coated by an outer protective layer of polymeric fucose derivatives. Such derivatives include, for example, the neoglycoproteins, fucosyl albumin and albumin fucosyl amines. Such protective coatings could be used to achieve semiselective targeting of tissue lesions following systemic intravenous administration of such composite carriers. By appropriate selection of the isoelectric and thermodynamic properties of these surface polymers, selective uncoating could be induced at sites of lowered pH which typically exist in microvessels which supply tumors and sites of chronic infection.

Selective uncoating is possible because glycoproteins and other surface polymers each exhibit their lowest solubility at their isoelectric point (pKI) and become increasingly soluble (unstable as surface coatings) as the pH is lowered below the pKI. Hence, the optimal isoelectric point for uncoating polymers in the body is at about blood pH (7.35). According to present art, the rate of such uncoating could be accelerated, for example, by incorporating a triggerable form of glucose oxidase in the microcarrier matrix which would generate gluconic acid and further protonate the surface polymer at lowered pH.

An important consideration in employing these technologies involves minimizing the rapid reticuloendothelial clearance of particles. Just recently, this has become feasible to accomplish by maintaining a small (ca. 50 nm) particle size and coating the particles with combination hydrophilic-hydrophobic block copolymers, such as the tetronic block copolymer 908™, the pluronic copolymer F68™ and others. A second method for inducing selective uncoating in lesional microvessels, is the use of surface coatings which are degraded by lesional degradative enzymes. These enzymes include serine esterases (e.g., tissue plasminogen activator and other enzymes of the coagulation cascade), and lysosomal enzymes (e.g., acid esterases and beta glucuronidase). A third method for selective uncoating involves the potential sensitivity of protective surfaces to external physical energy, such as occurs with melting of surface lipids by regional hyperthermia and disruption of hardened surface coatings by high-frequency ultrasound.

The endothelial envelopment-transport coatings documented below are adaptable for use with all synthetic and natural, solid (Matrix) and deformable (lipid and hollow) transvascular microcarriers, including microspheres, liposomes, artificial membranes, microvesicles, and hydrophilic and hydrophobic microemulsions, wherein the matrix and/or coating materials may be comprised of carbohydrates, oligo- or monosaccharides, proteins or peptides, lipids, alkyl or alkenyl chains, or bicompatible synthetic polymers. The drug or diagnostic agent carriers of the present invention may vary in complexity, including, for example:
1) single chain polymers;
2) molecular microaggregates in which the molecular carrier/aggregate comprises both the endothelial binding moiety and the backbone for linking prodrug moieties;
3) complex supramolecular carrier comprising multiple matrix material and/or serial coatings, with a major criterion of novelty being that multiple (two or more) endothelial binding sites are engaged by the carrier material or microcarrier surface in order to activate the endothelial cellular processes required for rapid envelopment (thereby sequestering the spheres from vascular degradation and drug from down-stream release during transendothelial migration) and transport of the carrier.

This invention is not considered to be constrained by prior art involving the formulation of microcarrier matrices from any of the presently proposed materials providing that the said materials were not previously recognized and documented in vivo as undergoing multiple endothelial binding and inducing rapid endothelial envelopment, and producing accelerated extravasation of macromolecules, microaggregates and microparticles in either the first microvascular bed encountered, or potentially (as proposed) semiselectively at foci of disease following systemic intravenous administration.

Endothelial-envelopment carriers may be formulated and stored in either the dry or fluid state, to which may be added, for example, pharmaceutically acceptable appropriate stabilizers, osmotic agents,colorings, flavorings, and physiologic solutions which render them appropriate for intravascular and intracavitary injection. The present invention is envisioned as most particularly applying to the vascular targeting phase of any future device (see below) which is developed for the efficient first-step transit across the external body barriers (e.g., gastrointestinal tract; oral, nasal rectal, bladder or vaginal mucosa; skin, cornea or sclera).

The present application documents that drug carriers which comprise microencapsulation spheres with surface adhesion properties were selectively taken up into tissues by endothelial bioadhesion and by induced transendothelial migration, into the tissue interstitium. The present application additionally documents that drugs controlled by such carriers, are deposited in selected target tissues, such as lung, in exact proportion to the deposition of drug carriers. It is now further established that soluble drug-carrier complexes (as well as formally microencapsulated drugs) give comparable tissue uptake of drugs, under conditions in which the drug alone is not taken up. It is now further established that the same and similar carriers are taken up by the transepithelial route in the lungs, gastrointestinal tract and bladder. Finally, it is established that the same and similar carriers undergo preferential lesional concentration in tumors and niduses of pulmonary infection.

The unique aspect of drug carrier technologies established by the present application are that these novel carriers afford high-efficiency tissue uptake and localization of drugs (and diagnostic agents) when the drugs are controlled by nonembolizing (less than 3 µm) carriers. Other unique features are that these carriers a) are formulated of water-soluble, biocompatible and biodegradable materials, and b) afford widespread percolation throughout tissue intersitium (and lesional gels) in a fashion which is not possible for hydrophobic carriers (e.g., liposomes). Finally, the carriers of principal embodiment interact with their initial sites of cellular uptake (endothelial and epithelial cells) based on carbohydrate-carbohydrate binding, as well as by protein-carbohydrate (and potentially peptide-carbohydrate and peptide-protein binding), and they do so in such a fashion as to produce multivalent binding, which leads to an induced, active endothelial (or epithelial) envelopment and transendothelial (or transepithelial) transport of both the carriers and drugs controlled by the carriers. This involves transcytosis (process occurring across one endothelial or epithelial cell, exclusively for the smaller less than 3 µm agent-carrier complexes).

The minimal requirements for novelty are that multivalent binding to cells (or adjacent matrix substances) must occur, in order to induce:
a) active extravasation (or epithelial transport) of the drug-carrier couple, wherein such transport is significantly accelerated relative to that obtained for uncoated (uncontrolled) particles or drug-carrier complexes; this acceleration being of such a degree that transcellular transport of nonembolizing as well as embolizing particles (complexes) is completed within twelve minutes of endothelial/epithelial contact (typically in less than 5 minutes), under in vivo conditions of microvascular blood flow and/or cavitary fluid flow, air flow, or enteric flow (in microvessels, bladder, lungs, bowel, or other body cavities, respectively) and further,
b) that the carriers must control the delivery of multiple (at least two) molecules of drug, in order to distinguish them from naturally transported simple hormones, proteins, peptides, and hybrid conjugates of two low-molecular-weight drugs.

The present continuation-in-part expands on examples initially provided and shows that certain of the drug-carrier systems (specifically, heparin-amphotericin microspheres) undergo not only primary uptake into normal tissues by accelerated transendothelial migration, but also secondary, subregional concentration within foci of disease involving the tissues (pulmonary infections; and pulmonary, hepatic, and subcutaneously implanted tumors). Such lesional concentration is based on:
a) the initial binding of heparin and heparin fragments to constitutive endothelial receptors (which comprise antithrombin III, thrombomodulin, heparin cofactor II, and others);
b) coincident or subsequent binding of heparin (or heparin fragments) to sites (tissue components) exposed (induced) by disease, comprising one or more of the following: epi-endothelial platelet factor 4 (and others), subendothelial fibronectin split products (e.g., 33,000 dalton proteolytic fragment), type IV collagen (and its subunits), type I collagen, laminin and others; and
c) finally, the active cellular uptake preferentially by transformed and malignant cells, of negatively-charged and neutral polycarbohydrates: specifically heparin, dextran and modified dextran microspheres which contained amphotericin B or heparin-cis-platin complexes. This cell biological uptake mechanism has been documented for hepatocellular carcinoma cells, and it putatively occurs for other transformed and malignant cells.

Although the preferred embodiment describes a surface coating of heparin, alternative carriers (and surface coatings and drug-complexing agents), such as heparin fragments, tridodecyl methylammonium chloride heparin, hereinafter referred to as TDMAC heparin, the dermatan sulfates and their fragments, and other glycosaminoglycans (GAG's), also serve to bind to constitutive and induced heparin cofactor II. The 8-12 unit fragment of dermatan sulfate binds heparin cofactor II without activating it. Unlike native heparin, neither dermatan sulfate nor its 8-12 unit fragment inhibits the constitutive endothelial surface coagulant, antithrombin III. This is also true of the shorter, semisynthetic fragments of heparin. Hence, dermatan sulfate and the short fragments of both heparin and dermatan sulfate, are envisioned as having even less anticoagulant activity than does native heparin (whose minimal anticoagulant activities are still acceptably low in this regard, when the heparin is incorporated into drug microspheres and complexes.

Additionally, endothelial and para-endothelial receptors are envisioned as being useful for selective organ uptake and secondary tissue localization in regions (foci) of disease. These include: the endothelial adhesion determinants induced by interleukin 1 (and by other cytokines and lymphokines), platelet activating factor(s) (PAF's), the surface coagulation factors, IIa, Va, VIIIa, IXa, Xa, XIa, von Willebrand factor (vWF), and endothelial tissue factor; and types IV and I collagen and the fibronectin fragments exposed by various disease processes (which can promote the attachment of metastatic tumor cells). Additionally, complementary substances are envisioned as useful in formulating surface coatings (complexes) for binding (and selective localization) at one or more of the preceding lesional sites. These include: peptide-11 (an anti-attachment substance for tumor cells); monoclonal antibody AHB-2 (and its Fab fragment) and fibronectin-binding polypeptide (the latter two of which bind the 33,000 dalton proteolytic fragment of fibronectin); agents which bind alternative fragments of fibronectin; flbronectin itself; fibronectin derivatives; and other complementary substrates; drugs; binding substances and their derivatives).

Epithelial uptake of the drug carriers (particularly the heparin-amphotericin B microspheres) are further tested in the present application and shown to be taken up via the intratracheal, gastrointestinal and intracystic (bladder) routes. Such epithelial uptake is tested for microspheres which comprise minor modifications of the drug carriers described in the present application. These formulations are as follows: microspheres with entrapped iron oxide, Fe₃O₄; and microspheres with heparin matrices, and heparin-coated dextran and albumin matrices, which contain entrapped ionic iron (Fe + 3). The novel examples of epithelial uptake shown below, provide the rationale for administering bioadhesion drug carriers by the intratracheal route (inhalation), gastrointestinal routes (oral and rectal), cystic route (bladder and prostrate), oral route with gastrointestinal uptake leading to systemic distribution (via the bloodstream) and to secondary targeting of the carriers to organs and lesions. These examples provide the further rationale for administering such drug carriers by injecting them directly into other body cavities, such as the peritoneum, uterine tubes, pleura, ventricules of the brain and spinal cord, epidural and subdural spaces, tumors and abscess, subcutaneous tissue, muscules, medullary cavities of bone, and joint spaces.

Endothelial uptake is described for a new physical formulation, namely a macromolecular complex between heparin and cis-platin (an antitumor drug). Selective high-efficiency pulmonary uptake of this drug and carrier complex is documented in the present application following bolus intravascular injection of the complexed agent, as is the absence of liver uptake, which typically occurs for the drug alone. The absence of endothelial injury by heparin-cis-platin (at an otherwise highly toxic concentration of 10 mg/ml cis-platin) is also documented. This novel result established the rationale for reformulating existing drugs using heparin and related kits (as devices), which can be performed by hospital pharmacists on-site, just prior to drug administration. This new approach can allow localized tissue (lesional) uptake of drugs controlled by nonembolizing carriers, as follows:
a) by intravenous administration to the lungs (high efficiency delivery) and systemic lesional sites (moderate efficiency delivery); or
b) by selective arterial perfusion to liver, kidney, brain, pelvis, extremities and other body sites (high efficiency delivery).

The present application describes that secondary tissue percolation of these hydrophylic drug-carriers occurs in normal target tissues for heparin-coated microspheres (interstitium, lymphatic and epithelial). In the present application, additional examples are presented, which establish the general principal that, unlike the situation for lipid microemulsions, liposomes and other hydrophobic carriers, the present hydrophylic spheres percolate extensively through the interstitium of a tumor and the lesional gel of a spontaneous pneumonitis, to reach both the outer spreading rims and the inner necrotic cores of these lesions. This provides new rationale for improved lesional penetration, cellular (microbial) access and uptake of drug carriers, and their entrapped (controlled) drugs. It is envisioned as allowing improved drug access to tumor cells and microorganisms lying in sequestered sites.

The present invention further specifies, that extensive percolation of interstitial tissue and lesional foci, can be achieved by pre-emulsifying hydrophobic drugs, such as amphotericin B, with a poloxamer (preferably, the pluronics, F108™ or F68™, but alternatively, the pluronics, F127™ or L61™, or the tetronic, 908™), which itself, percolates poorly, followed by microencapsulation of these controlled-release subparticles (nanoparticles or emulsions) in a larger, hydrophilic matrix carrier (e.g., of heparin) which itself, percolates extensively. Such formulations are novel and can be envisioned as artificial white cells. The outer, macromatrix performs the functions of the whole white cell (namely, bioadhesion to endothelium/epithelium, transport of the particles across their initial barriers and interstitial percolation); and the subparticles (internal nanoparticles, emulsions, or complexes) perform the function of the internal white-cell granules (namely, attachment to the final interstitial-matrix or cellular target and the controlled release of drug). These drug carriers are novel, because they represent multistage microparticles (complexes), with functionally-oriented surface coatings which govern both their initial (body) biodistributions and subsequent (local tissue) distributions. Their specification and testing establish a rationale for improving the biodistribution, localized uptake, tissue percolation, and cellular (or microbial)

### ACCESS

and uptake of drugs (and diagnostics) which do not normally accomplish these multiple steps on their own.

The original application of this series (Serial No. 033,432) and the present application further provide the novel rationale for secondarily controlling the release (or bioavailability) of drugs (diagnostics) from internally entrapped subparticles (nanoparticles, complexes or emulsion) of depot, time-release drugs (e.g. amphotericin B-pluronic F68™ and amphotericin B-cyclodextrin complexes, after these internally entrapped entities have been released (either rapidly or slowly) from the outer macromatrices of heparin and heparin-coated dextran. This is envisioned as allowing the additional flexibility of locking the internal microparticles into target tissues (cells) even if the outer matrix carrier subsequently redistributes from its initial target site.

Final drug uptake into rapidly growing or dividing cells is envisioned as being potentially augmented by formulating the internal (or external) drug particles with transferrin, ferritin, or anti-tumor antibodies (or antibody fragments) which themselves, may not percolate well into the most rapidly growing subregions of solid tumors (infections or abscesses). The improved gel percolation afforded by the present microcarriers is envisioned as improving the penetration of tumor glycocalyx, bony subcomponents of sarcomas, polyglucose hydrogels synthesized as attachment polymers by staphylococci and other organisms which cause osteomyelitis, periodontitis, and other bacterial infections; the proteinaceous microthrombi produced during invasive aspergillosis of the lungs and brain; the cartilaginous and ossified components of proliferative pannus which form in acute arthritis, and the gel substances which accompany other disease processes. Finally, these technologies are also envisioned as addressing the in vitro applications of penetrating cell-surface (cuticular) carbohydrates present on human and animal eggs and sperm, and on bacteria and yeast.

The present invention describes new entrapments of substances such as:
a) amphotericin B in lipid microemulsions coated with TDMAC heparin, which produces selective uptake in the lung following intravenous injection (and putatively in other organ/lesional sites, by the routes/methods specified above); and
b) the biomodulatory protein, interleukin 2, in albumin microspheres, which are amenable to coating with standard heparin, TDMAC heparin or heparin fragments, related glycosaminoglycans and their derivatives.

This provides the new rationale for heparin-modification and localization of previously hydrophobic carriers, such as microemulsions, liposomes, and microspheres of polylactic and polyglycolic acids. The present invention also envisions the selective localization of three new classes of biopharmaceuticals: recombinant proteins, peptides and DNA/RNA vectors (for reconstituting genetic disease). Finally, it envisions that this new drug carrier technology will be useful for site-specific transplantation of whole cell in vivo. Cell preparation for such targeting is envisioned as being performed using a bifunctional adhesive agent, one component of which (e.g., heparin, fibronectin, laminin, Fab fragments of monoclonal antibodies, etc.) binds noncycotoxically to the transplantable cells, and one component of which (potentially the same component) adheres multivalently to the target endothelium (epithelium). High-efficiency transplantation is envisioned as being achieved by introducing these cells by the direct vascular (or intracavitary) route leading to the target organ, tissue or cells.

The present invention envisions that the following diseases (and drugs) can potentially be treated (and localized) in an improved fashion by using the described technology: cancer (antitumor agents, biological response modifiers, particularly IL-2 and TNF, radiation sensitizers, perfluorinated hydrocarbons, and hyperthermia augmenting agents); prophylaxis against tumor and bacterial metastasis (depot heparin itself, the new antimetastatic peptide, peptide-11, etc.); diffuse infections and abscesses (antibiotic, antifungal and antiviral agents); deep pulmonary infections and central nervous system infections in immunocompromized and tumor patients (aminoglycoside and cephalosporin antibiotics, amphotericin B and other antifungals, and antivirals); chronic infection/inflammation of the urinary bladder or other sites (depot formulations of heparin, dermatan sulfate, or pentosanpolysulfates, gangliosides, haptens and peptide blockers, and their derivatives); multiple sclerosis; diabetic angiopathy, acute and chronic arthritis (copper chelating agents, such as penicillamine; steroids and steroid analogues), infarcts of the heart, brain, bowel and limbs (anti-adhesive agents which block neutrophil and platelet attachment, and free radical scavengers); disseminated intravascular coagulation (inhibitors of platelet activating factor, platelet adhesion and fibrin polymerization); atheroscelerosis (depot heparin, and heparin plus antilipidemic agents, such as probucol); acute coronary or cerebral thrombosis (tissue plasminogen activator -- administered intravenously or by selective arterial perfusion); genetic and degenerative diseases, particularly of the liver, pancreas and brain (appropriate degradative enzymes, pancreatic islet cells, pituitary and brain cells, and transfecting gene vectors); endometriosis (danazol, anti-inflammatory agents, and anti-vascular proliferative agents); infertility (sperm adhesives for in vivo and in vitro fertilization; prevention of allograft rejection, particularly for kidney, liver, bone marrow and lungs (steroids, immunosuppressives, cyclosporin A and others, antilymphocyte antibodies); acute and chronic asthma (depot antiasthmatic agents administered by inhalation); pulmonary emphysema, prophylaxis and treatment, and prevention of intercurrent lung damage from smoking (peptide and other blockers of neutrophil elastase, antiinflammatory agents, inhibitors of platelet activating factor, free radical scavengers, and their derivatives --administered by inhalation, orally, intravenously, or as additives to cigarettes, cigars or pipe tobacco).

The bioadhesion carriers set forth in the present application are envisioned as being preferred for the delivery of drugs which are highly toxic (certain antitumor drugs, antifungal agents, antibiotics, and many antivirals); drugs which are highly labile (peptides, hormones, recombinant protein biomodulators, and their analogues); agents which experience inappropriate biodistribution or poor tissue access due to their large molecular size or the presence of disseminated, competing receptors in the body (lymphokines, cytokines, interferons and other biologic response modifiers, and gene vectors); anti-adhesion pharmaceuticals (as depot formulations, for the prevention of cancer-cell metastasis, prophylaxis of atherosclerosis, and inhibition of white-cell and platelet adhesion to vascular endothelium); and most of the new, recombinant biopharmaceuticals, whose production costs may be extremely high, precluding administration in a freely circulating form (all of the new recombinant proteins, peptides and gene vectors, except in general, those which act directly on bone marrow).

Additionally, these new formulations and processes of facilitating cellular bioadhesion-uptake are applicable to cellular microinjections in vitro, including those of sperm, eggs, bacteria, yeast and others. Envisioned agents include high-efficiency injections of drugs, peptides, proteins, metals, diagnostic probes, transfecting gene vectors, mutational probes, whole sperm, sperm selected for sex preference by albumin gradient centrifugation, and other agents which need to be injected (ideally) under nonfusigenic conditions (e.g., conditions which avoid fusigenic viruses or chemicals, such as polyethylene glycol). This technology is envisioned as having implications for the fields of in vitro fertilization in both humans and animals, and for recombinant-gene transfections.

The following examples illustrate the invention described above.

### EXAMPLE 1

### Preparation of Acetone-Stabilized and Heat-Stabilized heparin Microspheres and Molecular Microaggregates

Beef lung heparin 100-200mg (152 units/mg, Upjohn Co.) was dissolved in 0.3-0.4 ml of distilled water and the solution emulsified in 6 ml of cottonseed oil (Sargent Welch, SC-11612™) by vigorous vortex mixing for 1 to 5 minutes. This initial emulsion was added dropwise into 19 ml of stirred cottonseed oil which had been preheated to 114-122°C. This suspension was maintained at high temperature for 10 minutes and then allowed to cool to room temperature with continued stirring. (Alternatively, the heparin suspension was added dropwise into an identical volume of stirred cottonseed oil at 22°C. The oil suspensions were added dropwise into 30 ml (5 times the oil volume) of a mixture of 0.1% Tween 80™ - (Sigma Chemical Co.) in acetone in order to extract the oil phase (and to produce stable crystallization of the heparin in the unheated preparation). The microsphere-acetone suspensions were centrifuged at 1,250 x g for 5 minutes to sediment the spheres. The microspheres were extracted an additional 3 times with 0.1% Tween 80™ in acetone (25 ml total volume or 4 times the oil volume). The resulting microspheres were either lyophilized to dryness or mixed thoroughly with 2% (w/v) Tween 80™ in 0.5 ml of acetone and allowed to air dry for 24 hours at 22°C. Both procedures gave heparin microspheres which were stable upon suspension in water or isotonic saline and had an average particle diameter of between 7 and 50 micrometers (µm) as measured by light microscopy. The sizes depended on the duration of vortex mixing in the oil emulsification steps above.

Heparin microaggregates averaging 0.1 to 0.2 µm in size were produced as described in the preceding steps, but with the addition of by sonicating the initial 6 ml of oil emulsion for 5 minutes at 20,000Hz with a standard ultrasonifier and special microtip (Heat Systems, Inc.).

### EXAMPLE 2

### Preparation of Heparin Microspheres Containing Entrapped Amphotericin B

### a. Entrapment of amphotericin-cyclodextrin complex.

Amphotericin B, 20 mg without deoxycholate (E.R. Squibb and Sons, Inc.) and gamma cyclodextrin, 31 mg (Polysciences, Inc.) were dissolved at a 1:1 molar ratio in 0.4 ml of dimethyl sulfoxide (Sigma Chemical Co.). Beef lung heparin, 49 mg (as in Example 1) was dissolved in 0.8 ml of distilled water. The two solutions were mixed and then rapidly emulsified in 6 ml of cottonseed oil by vigorous and continuous vortex mixing. Aliquots were removed quickly (due to partial but controllable phase separation of the drug-cyclodextrin complex) and added dropwise to 0.1% Tween 80™ in acetone according to the exact procedures described for the nonheated microsphere preparation of Example 1. The percentage of starting drug entrapped was 70% and the final drug content in spheres was 14% (w/w). Resuspension in water and isotonic saline resulted in two size populations of particles, the major fraction (ca. 85% by mass) comprised larger microspheres, 7 to 25 µm in diameter, and the smaller fraction (ca. 15% by mass) comprised smaller microspheres, 0.3 to 1.0 µm in diameter. These two fractions were rapidly separable by micropore filtration. The larger spheres were observed microscopically to be packed with yellow-colored refractile granules which were similar in size to the smaller particles just described. Water suspensions of the lypohilized spheres (combined size fractions) were amenable to complete sedimentation by centrifugation. By colorometric assessment of the fraction of amphotericin B (yellow color) which sedimented with particles at incremental times after aqueous resuspension, the t 1/2 of controlled release for amphotericin B was approximately 3 days.

### b. Entrapment of amphotericin B pre-emulsified with pluronic F68™ block copolymer.

Native amphotericin B, 100 mg without deoxycholate (E.R. Squibb and Sons, Inc.) and 12 mg of the pluronic F68™ block copolymer (polyoxypropylene-polyoxyethylene, Green Cross Corp.) were suspended in 1 ml of distilled water and ultrasonified for 1 minute (as in Example 1) to produce an initial emulsion with a particle size ranging from 0.1 to 5 µm in diameter. This suspension was stirred overnight at 22°C in the dark, and then ultrasonified for an additional 1 minute. The resulting emulsion was significantly smaller, with a particle size ranging from 0.1 to 0.8 µm in diameter. This emulsion was centrifuged at 500 x g for 2 minutes in order to sediment the larger (potentially uncoated) drug particles. The supernatant (fine emulsion, ca. 30-50% of the mass) was removed and used for subsequent entrapment in heparin microspheres. This was done by adding 70 mg of beef lung heparin (Upjohn Co., as in Example 1) to the 0.9 ml of recoverable supernatant (fine emulsion) stirring for 5 minutes to obtain complete solvation of the heparin, adding the resulting mixture to oil (preferably at room temperature, alternatively at 114-125°C for 10 minutes, for extra stabilization), emulsifying it by vortexing, quickly stabilizing the emulsion by stirring into 0.1% Tween 80™ in acetone at 22°C, and processing as described in Example 1. The resulting microspheres had an average diameter of 3-15 µm depending on the duration of vortex mixing. As assessed colorimetrically, the percentage of drug entrapped was greater than 70% and the final drug content was 20-30% (w/w).

Parallel microspheres were made as described above, except with dextran T70™ (Pharmacia Fine Chemicals) as the major matrix component and heparin as the surface coating (10% by weight). For these spheres, the surface coating was added as described in Example 3 below (starting at the text position marked "SURFACE COATING."

### EXAMPLE 3

### Preparation of Dextran T70 Microspheres with a Heparin Surface Coating of 10% by Weight

Amphotericin B, 20 mg without deoxycholate (E.R. Squibb and Sons, Inc.) and gamma cyclodextrin, 30 mg were dissolved in 0.4 ml of dimethyl sulfoxide (Sigma Chemical Co.). Dextran T70™ (Pharmacia Fine Chemicals), 49 mg was dissolved separately in 0.175 ml of dimethyl sulfoxide. The two aqueous suspensions were mixed and quickly emulsified in 7 ml of cottonseed oil (Sargent Welch, SC11612™). This oil suspension was added rapidly but dropwise to 0.1% Tween 80™ in acetone (T-Ac), 35 ml. Microspheres were sedimented at 1250 x g for 5 minutes. The pellet was extracted one additional time with 10 ml of 0.1% T-Ac, resuspended in 0.5 cc of 2% T-Ac and allowed to dry for 45-60 minutes at 22°C (until the acetone odor was no longer detectible). A SURFACE COATING was prepared as follows: Beef lung heparin (Upjohn Co., as above), 10 mg predissolved in 0.5 cc of distilled water, was added to the dried spheres. To this was added 6 ml of cottonseed oil (12 times the volume of water), and the suspension was emulsified by moderate vortex mixing, in order to apply the heparin coating to the surfaces of the previously crystallized dextran spheres. This emulsion was once again stabilized by dropwise addition to 30 ml of stirred 0.1% T-Ac, and the microspheres sedimented at 1250 x g for 5 minutes. Three additional extractions were performed with 10, 9, and 6 ml, respectively, of T-Ac. The pellet was resuspended in 0.5 ml of 2% T-Ac and allowed to air dry for 16 hours at 22°C. The percentage of drug entrapped was 65% and the final drug content was 12% by weight. Microsphere sizes ranged from 0.5 µm to 30 µm, depending on the duration of vortex mixing.

### EXAMPLE 4

### In Vitro Modification of Ulex Europaeus I Lectin Bound to Agarose Spheres

Ulex Europaeus I Lectin with affinity to endothelial factor VIII antigen, was obtained commercially (Vector Laboratories, Burlingame, CA) as a gel suspension in which the Ulex lectin was bound by a stable ether linkage, to agarose spheres (25-75 µm in diameter) of the lightly cross-linked polysaccharide comprising galactose plus 3,6-anhydrogalactose monomers). As obtained, the binding capacity was 2.5 mg of fucosyl glycoprotein per cc of gel and the suspension contained 10 mM fucose, the sugar hapten of highest specificity to saturate all Ulex binding sites.

### a. Preparation for injection of spheres with hapten-blocked (fucose-bound) binding sites.

To 0.25 ml of the unwashed gel was added 0.75 ml of 0.2M phosphate-buffered 0.15M(N) saline (Grand Island Biological Co.), in order to obtain a gel suspension which was sufficiently dilute for direct intravenous injection (below).

### b. Preparation for injection of spheres with unblocked (available) binding sites.

The gel, 0.25 ml was washed 3 times by centrifugation at 2500 x g with 0.8 ml each of 0.02 M phosphate-buffered 0.15M(N) saline, in order to remove almost all of the fucose sugar hapten which was initially bound to the Ulex binding lectin. The resulting pellet of spheres was suspended in a total volume of 0.8 ml for subsequent intravenous injection (below).

### EXAMPLE 5

### In Vivo Injection of Heparin Microspheres and Microaggregates Prepared as in Example 1

For all in vivo tests (this Example and Example 6 below), microspheres were suspended in phosphate-buffered saline (per Example 4) at a density such that their packed (centrifuged) volumes were 20 percent of their final volumes in suspension (spheres plus solution). Equivalent doses were given to each animal by injecting 0.125 ml of the fully suspended material. Lung targeting was accomplished by intravenous injection into CBA mice, and brain targeting was performed by carotid arterial injection into Sprague-Dawley rats. Analysis of organ targeting, envelopment and extravascular migration of spheres were carried out by 1) sacrificing representative test animals at 2, 5, 10, 15 and 20 minutes postinjection; fixing the brain tissue in 10% buffered formalin or inflating their lungs to a fixed size by injecting 10% Carson's buffered (pH 7.4) formalin intratracheally at a pressure equivalent to a 20-cm column of water; 2) processing the fixed tissue sections for light and electron microscopy; 3) staining these sections with hematoxylin and eosin (H & E), periodic acid Schiff (PAS), and reticulin histochemical stains; cutting (with a microtome) the light microscopic sections (below) at a 4-µm thickness; and 5) analyzing morphometrically, the processed sections for the number and microscopic position of spheres in relation to vessels, perivascular structures, interstitium and airspaces of lung, and the microvessels pericyte (astrocyte) processes (which abut the microvessels of brain), and brain tissue proper.

The legend for all Figures of tissue sections shown herein are: M = microsphere; V = microvessel; A = airspace; e = endothelial membrane; and n = endothelial nucleus.

### a. Injection of heparin microspheres (0.125 ml) intravenously and localization in CBA mouse lung.

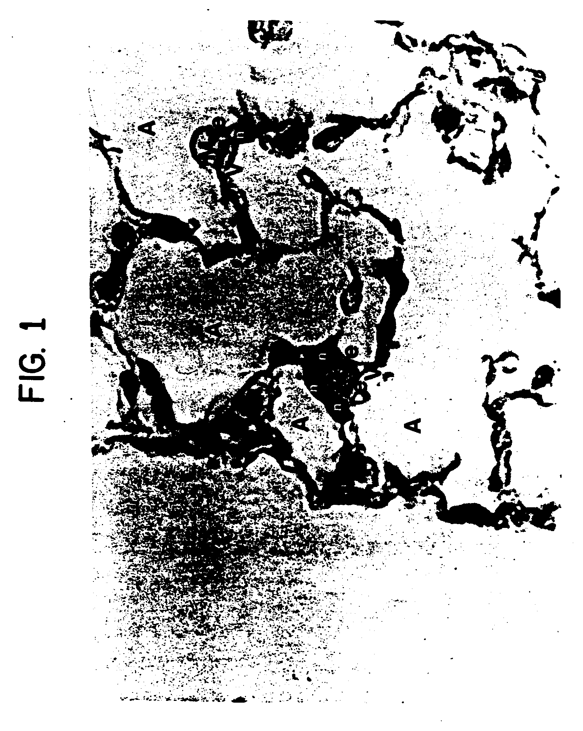

Figure 1 is a lung tissue section stained with PAS, which is representative of the test mice sacrificed 2-5 minutes after intravenous injection of the unheated, acetone-stabilized heparin microspheres of Example 1. At the center is a typical heparin microsphere (M) approximately 20 µm in diameter, which has become lodged within the microvascular lumen of a lung capillary and is already completely enveloped by endothelial cell membrane (e), whose two nuclei (n) are present immediately adjacent and overlying the sphere. At the upper right-hand corner is an endothelial-coated microsphere (M) which has migrated partially out of its lung capillary (V) and is beginning to lose its endothelial coating (e, at 4-6 o'clock on the sphere) at position 8-9 o'clock on the sphere.

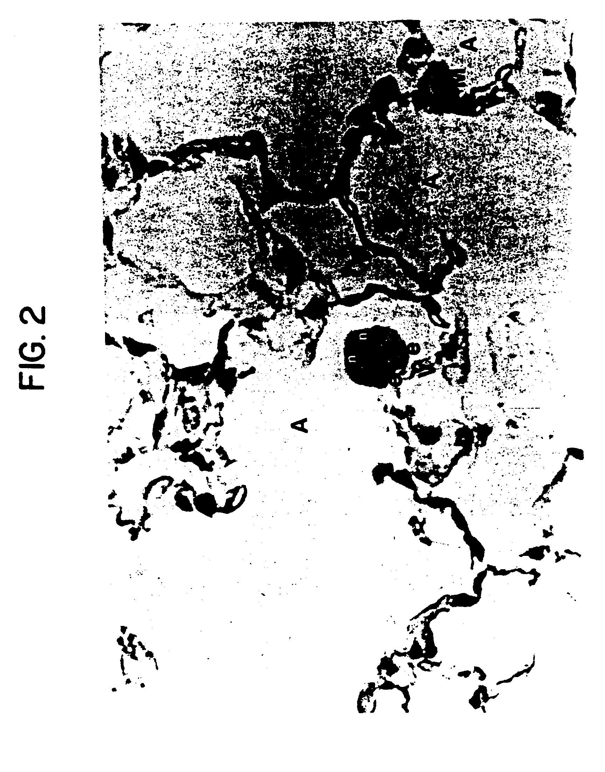

Figure 2 is a lung tissue section stained with PAS, which is representative of the test mice sacrificed 10 minutes after intravenous injection of the same heparin microspheres as in Figure 1. At center is microsphere (M) the same heparin microspheres as in Figure 1. At center is microsphere (M) which has migrated almost completely out of its lung capillary (V) into the adjacent airspace (A). Endothelial membrane (e) and nuclei (n) are still present on the microsphere surface. There is minimal to no toxicity to the microvessel as evidenced by an absence of co-extravasted red blood cells or serum proteins (which would stain intensely with PAS). A second endothelial-coated and partially extravascular microsphere is present at lower right.

Smaller (nonembolizing) microspheres and microaggregates of all the heparin and heparin-coated formulations of Example 1 are observed to undergo similar envelopment and extravascular migration at approximately the same kinetics.

Table 1 summarizes the percentages and positions of intrapulmonary microspheres of 4 to 15-µm diameters 15-20 minutes after intravenous injection:

**Table 1**

| Type of sphere | Approximate percentage of injected dose identified in lung | Percentage of spheres in extravascular locations |
|---|---|---|
| 1. Heparin (acetone) | 35 | 85 |
| 2. Heparin (heated) | 40 | 80 |
| 3. Plain agarose* | 10 | 20 |

| | | |
|---|---|---|
| *Many of the remanent intravascular spheres were undergoing degradation due to serum amylase digestion, and only small fragments of these spheres could be identified. | | |

These histologic and morphometric results document that the heparin microsphere surfaces induce rapid (less than 2 minutes) partial and/or complete endothelial coating which resulted in endothelial envelopment (walling-off) of the spheres, thereby functionally removing them from the vascular compartment (even during before they emigrate out of the vascular space). This slows intravascular degradation of the spheres and accelerates extravascular migration of the intact spheres (largely complete within 15 to 20 minutes), and greatly increases the proportion of spheres which become localized in the tissue (interstitial) compartment and airways

Larger heparin microspheres (25-75 µm diameters) experience pulmonary captures and extravascular migrations similar to those of the Ulex I spheres shown in Table 2 of Example 6, below.

### b. Injection of heparin microspheres into the carotid artery and localization in Sprague-Dawley rat brain.

Heparin microspheres from Example 1 (0.250 ml, 5-15 µm in diameter) were injected into the carotid artery and the rats sacrificed at 15 minutes. One to seven, small (0.2-3.0) PAS-positive particles were observed in and surrounding the microvessels of the cerebral and cerebellar cortex and the deep nuclei of the brain. Approximately 50% of the vessels were positive for emigrating particles. At 15 minutes postinjection, these particles were present largely along the processes of pericytes lying adjacent to the brain arterioles and capillaries. (Pericytes are thought to be involved in the transport of nutrients from the vessels into brain parenchma.) Smaller numbers of PAS-positive particles were identified at greater distances away from pericytes within the extracellular compartment of brain tissue proper. Morphometrically, at least 15 percent of the injected microspheres were localized in brain tissue at 15 minutes.

### EXAMPLE 6

### In Vivo Injection of Ulex Europaeus I Lectin Microspheres Prepared in Example 1

Ulex Europaeus I lectin microspheres (0.125 ml) were injected intravenously for localization in CBA mouse lung.

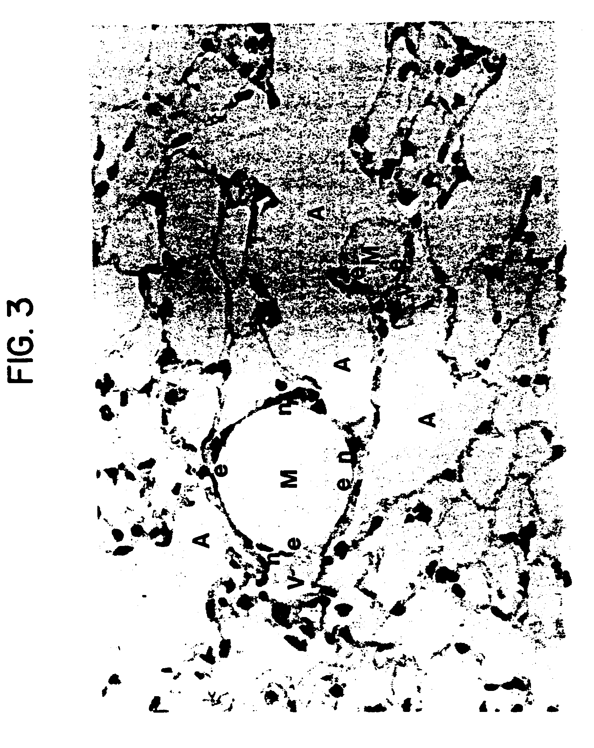

Figure 3 is a lung tissue section stained with PAS, which is representative of the test mice sacrificed 2-5 minutes after intravenous injection of the fucose-blocked, Ulex Europaeus agglutinin I-coated spheres of Example 4. A larger microsphere (M) is present (at left center) in the vascular space (V), which has undergone almost complete envelopment by endothelial membranes (e) and nuclei (n). A smaller microsphere (M) is present (at right center) which has undergone both endothelial envelopment and almost complete extravascular migration into the airspace (A). However, it remains attached to the basement membrane of the small vessel from which it emigrated. Remnants of endothelial membrane (e) still coat it at the surface of attachment but have been lost from the free surface. Histologic comparisons of heparin and Ulex I microspheres have revealed that a higher proportion of emigrated Ulex I spheres remain attached to the abluminal basement membrane, whereas a higher proportion of the heparin spheres (Example 5 above) have further migrated into distant structures, including lymphatics and airways. For all spheres, there was an absence of red blood cell attachment on the downstream surface, indicating that any tendency towards binding or agglutination of red cell surface blood-group substances had been successfully blocked by the sugar hapten Also, there was histologic evidence for the induction of acute coagulopathies or endothelial toxicity.

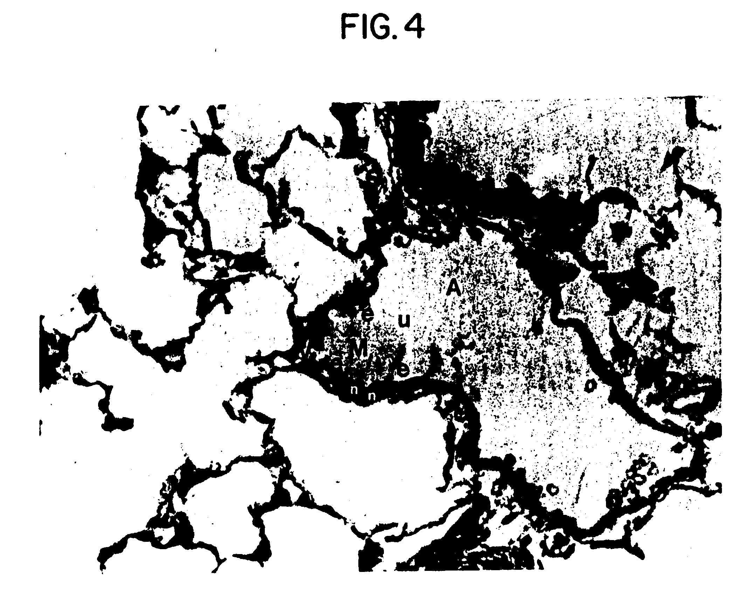

Figure 4 is a lung tissue section stained with a reticulin stain, which is representative of the test mice sacrificed at 10 minutes after intravenous injection of the identical fucose-blocked, Ulex Europaeus agglutinini-coated spheres of Example 3. At center, is a microsphere (M) which has undergone complete emigration from the vascular space (V) into the airspace (A), with continued attachment to the abluminal basement membrane. This sphere shows remanent coating by endothelial membranes (e,e) but uncoating on the opposite surface (u). Small fragments of reticulin (a connective tissue component of the vessel wall) have been carried through into the airspace with the microsphere (dark stringy material just below "A") but no red blood cells have been released from the vessels into the airspace. (Emigration of reticulin is not observed with emigration of the smallest, 10-µm spheres present in this Ulex I suspension.) The microsphere of Figure 4 is beginning to undergo degradation in the airspace at 10 minutes. At 20 minutes, the extent of degradation is only slightly greater that at 10 minutes for most of the extravastated sphere matrices (not shown). Examples 3 and 4 indicate that fucose-blocked Ulex I spheres undergo efficient uncoating upon contact with endothelial surfaces which have binding sites for the Ulex I lectin, and that this event induces endothelial envelopment and rapid extravascular migration of the spheres. Similar responses are seen for unblocked microspheres (with exposed Ulex I binding sites.) For smaller (nonembolizing) Ulex I spheres of 3-5 µm diameters, such uncoating would be expected to occur preferentially in the microvessels supplying focal lesional tissues (involved by inflammation, infection and tumor).

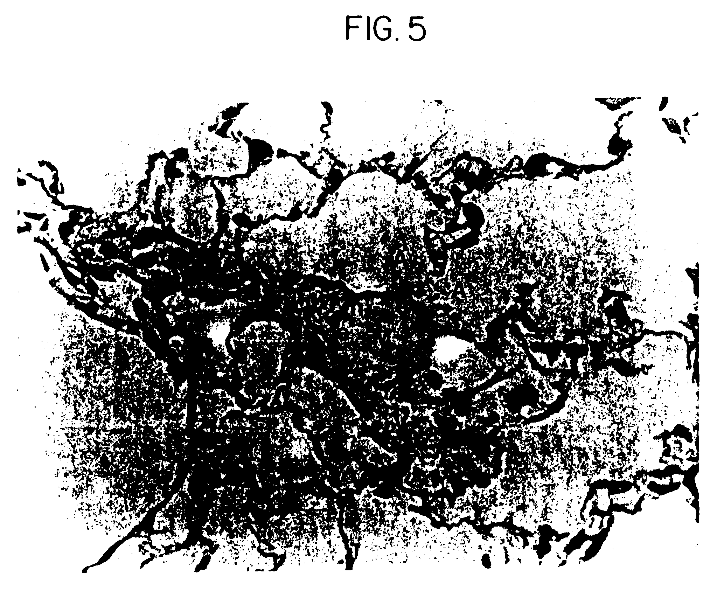

Figure 5 is a lung tissue section stained with PAS, which is representative of the test mice sacrificed 20 minutes after intravenous injection of the identical fucose-blocked spheres of Examples 3 and 4. This exemplifies the rare intravascular microsphere (M) which can still be identified at 20 minutes. Although it has undergone nearly complete endothelial envelopment and partial extravascular migration, its migration is not yet complete. This rare example shows that the portion of the sphere which is most completely coated by endothelial membranes (e) is the most protected from intravascular amylase digestion and remains morphologically intact. Conversely, the portion of the sphere which is uncoated (the portion which invaginates most deeply into the vascular compartment "V") is has undergone morphologic fragmentation (f) and will shortly become completely digested within the vessel unless it first completes the process of emigration. This indicates that endothelial envelopment indeed renders the emigrating particles extravascular and hence protects them from digestion during the process of emigration. BY the same process of walling off the particle, it can be inferred that most of the drug which is released in this newly formed endothelial pocket during microsphere emigration would also be walled off and released into the tissue compartment as the particle emerges on the tissue side. Note that blood flow has already been reestablished in this vessel at positions 5-7 o'clock around this sphere.

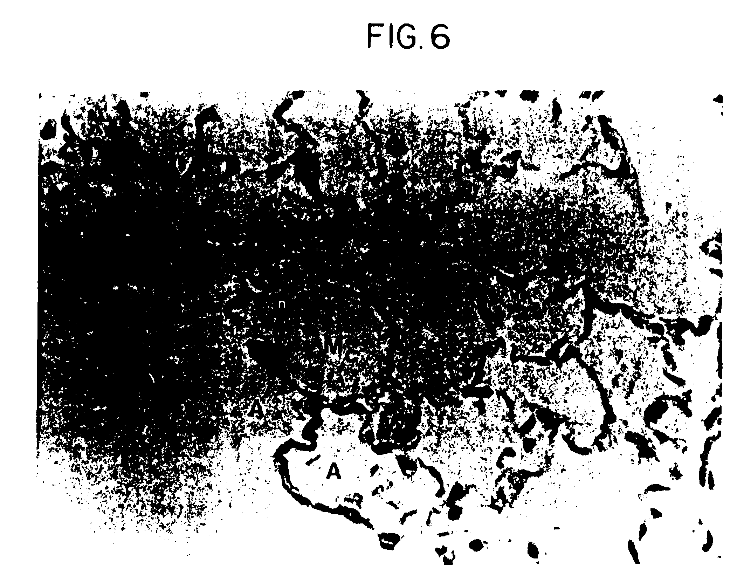

Figure 6 is a representative example of control microsphere (M_{c}) of plain agarose which is present within a lung microvessel (V) 10 minutes after intravenous injection. In contrast to the Ulex I (and heparin) spheres, this sphere shows no evidence of endothelial coating on either the upstream or downstream free surfaces (u, uncoated). It also shows no evidence of beginning extravascular migration. A reticulin stain (not shown) indicates intact reticulin around all aspects of the vessel wall with which the sphere is in contact. Such control spheres (without Ulex I or heparin surfaces) migrate in a delayed (20 minutes or longer) inefficient manner (see Table 2 below), and undergo intravascular degradation with downstream release of microsphere fragments and drug.

Table 2 summarizes the percentage and position of intrapulmonary microspheres of 25 to 75-µm diameters at 10-20 minutes after intravenous injection:

**Table 2**

| Type of sphere | Approximate percentage of injected dose identified in lung | Percentage of spheres in extravascular locations |
|---|---|---|
| 1. Ulex I, fucose-blocked* | 90 | 80 |
| 2. Ulex I, unblocked* | 90 | 90 |
| 3. Plain agarose ** | 10 | 20 |

| | | |
|---|---|---|
| *The higher lung-capture percentage of Ulex I versus the heparin spheres of Example 5, Table 1, is due to the larger diameters of these particles. Note, however, that plain agarose particles of the larger diameter (Table 2) are not effectively transported into the tissues, and hence, their capture percentage at 10-20 minutes is also low due to intravascular degradation and release from the lung. Smaller spheres with Ulex I surfaces would be expected to undergo capture percentages equivalent to heparin spheres of the same size. | | |
| **Many of the remanent intravascular spheres were undergoing degradation due to serum amylase digestion, and only small fragments of these spheres could be identified. | | |

### EXAMPLE 7

### Prophetic Formulations of Preferred and Alternative Embodiments.

The following embodiments continue and augment those presented heretofore and include
1. Microparticles expanded to include nanoparticles and molecular complexes of diameters and Stokes radii down to 0.02 nanometers.
2. Additional matrix materials envisioned as follows:
   a) preferably, Type IV collagen or albumins, coated with multivalent heparins or TDMAC heparin, respectively;
   b) alternatively, lisinopril-albumin conjugates, polylactic acids, polyglycolic acids, mixtures of polylactic and polyglycolic acids in crystalline and/or amorphous forms, starch, hydroxyethyl starch, and other starch derivatives, oil-in-water emulsions, liposomes, laminin (and its fragments), type I collagen (and its subunits), fibronectin (and its fragments), antithrombin III (and its binding subunits);
   c) For paired-ion complexation and stabilization of negatively charged matrix materials:
      (1) preferably, protamine or polyethyleneimine (PEI);
      (2) alternatively, poly-L-lysine, or other positively charged amino acids or intermediate metabolites, bacterial or recombinant products, or their derivatives, for which, in order to minimize in vivo -(particularly endothelial) toxicity, the positively charged moieties are located predominantly internally and the negatively charged moieties are located predominantly on the external surfaces;
   d) For semiselective tumor-cell uptake, inclusion of negatively charged or neutral polycarbohydrates;
      (1) preferably, the sulfated polyglucose, heparin, or neutral polyglucose, dextran, as specified initially in the parent document (Serial No. 033,432);
      (2) alternatively, dermatan sulfate, pentosanpolysulfate or starch, and their derivatives.
3. Additional surface coatings and drug complexing substances are specified as follows:
   a) preferably, low-molecular-weight fragments of dermatan sulfates;
   b) alternatively, lisinopril; lisinopril conjugates of dextran, starch, starch derivatives or albumin; negatively charged glycolipids or gangliosides and their derivatives, TDMAC heparin (or other hydrophobically modified heparins or glycosaminoglycans, GAG's) in which the hydrophobic substituent is attached to the heparin (or GAG) by either covalent conjugation or paired-ion complexation (particularly for use in coating matrices which are hydrophobic or have hydrophobic subregions, such as denatured albumins, polylactic and polyglycolic acids, oil-in-water emulsions, and liposomes);
   c) For targeting cellular and/or matrix heparin sulfates or heparin glycosaminoglycans in vivo;
      (1) preferably, type IV collagen;
      (2) alternatively, antithrombin III, recombinant heparin cofactor II, 33,000 dalton proteolytic fragment of fibronectin, and laminin;
   d) For targeting tissue matrix components exposed by disease processes in vivo:
      (1) preferably, type IV collagen;
      (2) alternatively, laminin, antithrombin III, recombinant heparin cofactor II, fibronectin-binding polypeptide, Fab fragments of AHB-2 monoclonal antibody (directed against the 33,000 dalton proteolytic fragment of fibronectin) fibronectin, 33,000 dalton proteoyltic fragment of fibronectin;
   e) For targeting renal glomeruli and the kidneys:
      (1) preferably, the FDA-approved intravenous injectable, protamine;
      (2) alternatively, polyethyleneimine, poly-L-lysine or other positively charged amino acids or intermediary metabolites, bacterial or yeast-derived recombinant products, NH2-containing monomers, oligomers or polymers, or derivatives of the preceding compounds.
   f) For augmentation of gastrointestinal uptake:
      (1) preferably, sodium lauryl sulfate or dioctyl sodium sulfosuccinate;
      (2) alternatively, the alkyl aryl sulfate, G-3300, sodium taurocholate, or other biocompatible, conjugated, complexed, physically mixed or emulsified, sulfated and sulfonated detergents.
   g) For augmented binding to sites of intravascular coagulation/fibrinolysis:
      (1) preferably fibrinogen;
      (2) alternatively, Fab fragments of anti-fibrinogen antibodies, and reagents which bind to the activated surface coagulation factors, IIa, Va, VIIIa, IXa, XIa, endothelial surface phospholipids, vWF, etc.

      Any or all of the preceding surface coatings are envisioned as being applied to their underlying matrices either as a thick or thin (monomolecular) film, and in a single-fluid phase, fluid-emulsion, or by microparticulate suspension methods, which include air suspension, ultrasonic suspension in vacuum, and which are accomplished by one or more of the following physical or chemical means;
      a. hot-plasma coating;
      b. cold-plasma coating;
      c. vacuum sputtering;
      d. fluid-phase transfer (direct deposition or reverse emulsification);
      e. particle-surface activation by tresyl chloride, cyanogen bromide or alternative chemical reagents, followed by covalent bonding of receptor-binding peptides or proteins;
      f. particle-surface activation by periodate oxidation of vicinal hydroxyl groups, followed by covalent bonding, by ado condensation, of carbohydrates, dextrins, glycoproteins, or glycolipids; or, by amino condensation, of proteins and peptides, with subsequent reduction with borohydrides;
      g. bonding of internal and external particles (or emulsions, particle-emulsion hybrids, or glycosaminoglycan-detergent complexes or covalent conjugates) by any of the previous methods;
      h. for the cases in which either the particulate matrices or pharmaceutical-complexing materials (e.g., type IV collagen, laminin, antithrombin III, and fibronectin--for heparin; and/or other glycosaminoglycans, peptides, proteins, etc.), have native binding sites for the surface coating materials, direct addition of the preformed particles (or complexes) to the complementary surface material, such that the surface coating (complexing substance) binds the matrix by noncovalent association, and leaves additional (identical or alternative) free binding groups (preferably of heparin or type IV collagen; alternatively of laminin, dermatan sulfate, or other glycosaminoglycans) available on the external surfaces for endothelial or epithelial adhesion); also, in cases where such matrix-binding materials are not natively multivalent, a procedure for polymerizing or complexing the haptenic subunits together, such that they are reformulated to become multivalent, and such that these synthetic multimers can bind both internally, to the underlying matrix, and externally, to their biological targets: endothelium, epithelium and/or extracellular matrix components.
4. The present invention envisions formulations which employ additional detergents as excipients for preparing the internal drug nanoparticles, nanoemulsions, or other internally entrapped, controlled-release subcapsules, complexes or agents (as was described initially in the parent document (Serial No. 033,432) for formulation and entrapment of the internal drug emulsion, amphotericin B-pluronic F68™. Such detergents include:
   a) preferably, sodium deoxycholate;
   b) alternatively, cholesterol, Tween 80™, zwitterionic detergents, or other biocompatible nonionic, polysulfated or positively charged detergents, as needed to formulate stable drug emulsions.
5. The present invention envisions the use of additional methods for matrix stabilizing and controlling the release of drugs. These include:
   a) preferably, for proteins and heat-labile peptides, chemical cross-linking with fresh formaldehyde or paraformaldehyde;
   b) alternatively; addition of thickening agents, such as polylactic and polyglycolic acids, polyaminoacids, poly-L-lysine, polyethyleneimine, glycerol, polyglycerols or polyalchols (with or without heating or chemical reaction), polyethylene oxides, biodegradable poloxamers or poloxamines (pluronics or tetronics), poly-COOH compounds (polycarbols), or polyamines.
6. Additional methods of microparticle formulation are envisioned as including (particularly for the purposes of product scale-up):
   a) preferably, extrusion of matrix (and/or surface) components through single (and/or coaxial), sonified or air-stream-fractured micro-orifices (single or multiport);
   b) alternatively, aerosolization using hybrid, homogenization-spray drying apparatus.
7. The present invention envisions additional methods of extracting the solvents used for phase emulsification and simultaneously crystallizing the matrices, surfaces and/or entrapment materials):
   a) preferably, hexanes;
   b) alternatively, ethanol or methanol.
8. Additional methods of sterilization (and/or particle sizing) of the final (or subfinal) preparations, include:
   a) preferably, for heat-stabile agents: autoclaving at 120°C for 10-20 minutes;
   b) preferably, for heat-labile agents:
      (1) submicron filtration of complexes and nanoparticles; and
      (2) irradiation of particles larger than 0.22 um;
   c) alternatively, ultrasonification.

### EXAMPLE 8

### Formulation of controlled-release, heparin-amphotericin B-pluronic-F108™ drug particles

Two formulations of heat-stabilized, heparin-amphotericin B microspheres were prepared as described in Example 2.b. with the following modifications: for pre-emulsification of amphotericin B (E.R. Squibb and Sons, Inc., Princeton, NJ), the pluronic detergent, F108™ (BASF Corp., Parsippany, NJ) was substituted for pluronic F68™; heat stabilization of the drug matrix was carried out at 115°C for 10 minutes with continuous shearing using a high-speed sonicating probe-homogenizer (Brinkmann Instruments, Westbury, NY); and two fractions of spheres with different diameters were harvested by centrifugation of the larger ones at 250 x g for 15 seconds, in acetone + 0.1% (w/v) Tween 80™, followed by complete sedimentation of the smaller ones which initially remained suspended. This produced two size fractions of amphotericin-B entrapment spheres, each of which comprised 50% of the total product weight.
a) The smaller fraction comprised nanospheres, which ranged from 150-700 nanometers (nm) in diameter. This nanosphere formulation contained 33.6% (w/w) amphotericin B, as determined by extraction in dimethylsulfoxide:methanol (10:90) and reverse-phase (C18) high performance liquid chromatographic (HPLC) analysis of extracted amphotericin B. Upon aqueous suspension of the nanospheres in intravenous injection solution, 5.3% of the total drug (= the surface fraction) was released rapidly (over 15 minutes). The remaining drug, 94.7%, was released in a controlled fashion, with a t1/2 of 24 hours.
b) The larger fraction comprised microspheres, which ranged from 1-8 micrometers (µm) in diameter. This microsphere formulation contained 41.7% amphotericin B (w/w), 2.7% of which was released rapidly, and 97.3% of which was released in a controlled fashion, with a t1/2 of 32 hours.

### EXAMPLE 9

### Testing the pulmonary and extrapulmonary localization of intravenously administered heparin-amphotericin B-F108 nanosphere and microsphere formulations prepared as in Example 8

Organ localization of the amphotericin B formulations prepared as in Example 8, was tested by intravenous injection into adult male CBA/J mice. Drug concentrations were analyzed both chemically and histologically. Chemical analyses were performed by sacrificing the animals at 10 min to 6 hrs postinjection and performing tissue homogenization, drug extraction, and HPLC quantification of drug (as described for the microspheres, in Example 8, above). Histological analyses of amphotericin-poloxymer localization and tissue-subregion distribution were performed by sacrificing the animals at 15 minutes postinjection, and performing a special histochemical stain (extended oil red O) on the 10-micron thick sections, representative of all major body organs.

Histologic results revealed that carrier localization and uptake by endothelium at all anatomic sites, was nearly complete within 10 min and entirely complete at 15 min postinjection. By HPLC analysis, blood levels were at the limits of detection at 10 minutes and undetectible at 1 hour postinjection. Hence, the 1-hour chemical data (below) represent equilibrated biodistributions. The 1-hour results for both the nonembolizing nanospheres and marginally embolizing microspheres of Example 8, were compared with those of standard amphotericin B (Fungizone™ formulated as a simple amphotericin B-deoxycholate nanoemulsion (E.R. Squibb and Sons, Inc., Princeton, NJ). All doses of amphotericin B were maintained at 0.75 mg/kg of body weight.
(1) Biodistribution of nanoparticulate heparinamphotericin B-F108 at 1 hour postinjection:
   (a) Total body drug recovered 70%;
   (b) Organ concentrations (µg amphotericin/gram tissue, wet weight): lungs 15, liver 8.3, kidneys 1.1, spleen 6.2, brain 0.20, heart 1.4;
   (c) Percentage of injected dose localized per gram of tissue (wet weight): lungs 52, liver 29, kidneys 3.8, spleen 21, brain 0.7, heart 4.8.
(2) Biodistribution of microparticulate heparinamphotericin B-F108 at 1 hour postinjection:
   (a) Total body drug recovered 55%;
   (b) Organ concentrations (µg amphotericin/gram tissue, wet weight): lungs 25.7, liver 5.0, kidneys 0.4, spleen 2.2, heart 0.3, brain
   (c) Percentage of injected dose localized per gram of tissue (wet weight): lungs 94, liver 18, kidney 1.4, spleen 8.2, heart 0.4, brain 0.5
(3) Biodistribution of Fungizone™ (amphotericin B-deoxycholate nanoemulsion) at 1 hour postinjection:
   (a) Total body drug recovered 58%;
   (b) Organ concentrations (ug amphotericin/gram tissue, wet weight): lungs 4.0, liver 6.7, kidneys 2.5, spleen 11, heart 0.3, brain 0.
   (c) Percentage of injected dose localized per gram of tissue (wet weight): lungs 14, liver 24, kidneys 9.2, spleen 38.3, heart 1.3, brain 0.

These results establish that preferential and rapid lung uptake of intravenously administered amphotericin B occurs when it is formulated as heat-stabilized heparin-F108 spheres of either subembolizing or embolizing diameters. These results of drug localization correlate exactly with the morphometric results of carrier localization, which were earlier obtained. As presented in the present application, the chemical uptake and anatomic subregion locations of amphotericin B entrapped in subembolizing nanospheres, further establish that the mechanism of uptake is as follows: endothelial bioadhesion, plus induced active endothelial envelopment and transport of the heparin-coated particles into the tissue interstitium. Further evidence for initial endothelial bioadhesion is provided by the observation that the 1-hour lung uptake of heparin-amphotericin B nanoparticulates is decreased by 52% if soluble heparin is injected at the same time as heparin-drug particles. Further evidence that active endothelial transport of amphotericin B is induced by the heparin surface, is provided by the combined results that lung sequestration of drug is nearly complete and blood levels nearly zero at the very early time of ten minutes following intravenous injecton. Such rapid and efficient uptake is not observed for dextran and agarose placebo particles which lack the heparin surface coating.

The quantity of amphotericin B which becomes deposited in the lung after one hour, also remains largely within that organ at extended times, as assessed both histologically and by delayed chemical analyses. By chemical analysis, the 6-hour retention of amphotericin B within the lungs of 6-month-old adult CBA/J mice was 60 to 70 percent of the 1-hour values. By histochemical staining, amphotericin B distributes widely within lung alveoli, pulmonary interstitium, respiratory epithelium, and bronchial/tracheal lymph nodes. This indicates that tissue percolation of the drug carrier is extensive, and that such percolation can provides wide coverage of both the primary tissue targets and the secondary, lymphatic drainage routes. In the occasional mice which had acquired focal pneumonitis (spontaneous mycoplasma infections), the density of nanospheres and microspheres was significantly higher in these foci of infection than in the surrounding normal tissues. This indicated that heparin nanospheres (and microspheres) have an additional advantage of secondary lesional accumulation (of amphotericin B). Hence, although extra-pulmonary redistribution occurred, its extent was minor, and importantly, the intra-pulmonary redistribution which also occurred, favored the selective accumulation and prolonged retention of drug in sites of infection (vis a vis normal tissue components).

An intentional superdose of (subembolizing heparin-amphotericin B-F-108 nanospheres was administered intravenously to a group of mice, in order to test the systemic organ uptake which occurred following supersaturation of lung receptors. This resulted in secondary organ clearance by the liver, however, the histologic pattern differed importantly from that usually observed with nonspecific (nonlocalizing) particulates in that these new heparin particles became localized within hepatocytes, as well as Kupffer cells. This established that the heparin particles experienced general endothelial-cell uptake rather than preferential phagocytic-cell uptake. The heparin-amphotericin B-F108 nanospheres also continued to avoid the kidneys (major site of amphotericin toxicity). These results, together with the previous results of brain uptake by selective carotid arterial perfusion (Example 5.b.), establish that it is also possible to achieve high-efficiency endothelial bioadhesion, selective drug uptake and retention at sites (organs) other than lung.

### EXAMPLE 10

### Formulation of a TDMAC heparin-coated lipid nanoemulsion containing amphotericin B

This nanoemulsion was produced by reformulating the FDA-approved lipid emulsion, 20% (w/v) Intralipid (KabiVitrum, Inc., Alameda, CA) by adding amphotericin B at 15% (w/w) to the emulsion, allowing it to stir into the (soybean) oil component of the emulsion for 20 minutes, then adding TDMAC heparin (Polysciences, Inc., Warrington, PA) at 0.2% (w/w) to the resulting composite, and sonicating this for 20 seconds, in order to accelerate the incorporation of TDMAC heparin into the egg-yolk phospholipid surface. The resulting composite lipid-emulsion particles ranged from 200 to 700 nanometers in diameter, and were stable (by inverted light-microscopic analysis) for longer than 2 hours (sufficient to permit controlled intravenous infusion).

### EXAMPLE 11

### Testing the pulmonary and extrapulmonary localization of intravenously administered TDMAC heparin-amphotericin B-lipid nanoemulsion prepared in Example 10

The TDMAC heparin-lipid nanoemulsion of amphotericin B (formulated as in Example 10) was injected intravenously into adult male CBA/J mice. At 1 hour postinjection, the lung-to-kidney ratio of amphotericin B content was increased significantly to 1.44:1 (relative to that observed for Fungizone™, = 1.17:1). Also, drug concentrations in the spleen, liver and kidney were reduced by multiples of 2.2 and 1.6, and 1.7, respectively, relative to those achieved by an equal dose of Fungizone™. This established the capacity of TDMAC heparin-lipid microemulsions of amphotericin B, to minimize the pronounced phagocytic uptake both by the reticuloendothelial organs/cells, as well as the clearance by kidneys (the organs of major toxicity).

Special histologic staining was performed on the frozen sections of all organs at twelve minutes postinjection. This confirmed moderate staining intensity and homogeneous distribution in lung alveoli, of the TDMAC heparin-lipid emulsion of amphotericin B. It further indicated that significantly less of the Fungizone™ emulsion became localized in the lung. These results indicate that heparin modification of nanoemulsions resulted in improved pulmonary localization of amphotericin B. Although this localization was less efficient than that afforded by stabilized nano- and microcarriers, it was still significantly more efficient than that afforded by a simple nanoemulsion (without heparin).

### EXAMPLE 12

### Formulation of a heparin-cisplatin, paired-ion emulsion complex

The FDA-approved antitumor drug cisplatin (Platinol™, cis-diaminodichloro platinum coordinate, Bristol Laboratories, Syracuse, NY) was reformulated as a metastable heparin complex, by rehydrating Platinol™ with distilled water at a concentration of 10 mg/ml, mixing the drug with beef lung heparin (Upjohn Co., Kalamazoo, MI) at weight ratios of 1:1.1 (cis-platin to heparin, w/w), and ultrasonifying for 1.5 min, in order to accelerate the formation of paired-ion complexes between the amino groups of cis-platin and the sulfate groups of heparin. This resulted in a heparin-coated cis-platin microemulsion complex, with particle dimensions of 0.2-1.5 um (cisplatin itself being insoluble at concentrations above ca. 2 mg/ml). The resulting microemulsion complex remained stable for longer than 1 hour at 22°C (sufficient for controlled intravascular infusion).

### EXAMPLE 13

### Testing the pulmonary and extrapulmonary localization of intravenously administered heparin-cis-platin, paired-ion emulsion complex prepared in Example 12

The heparin-cis-platin microemulsion complex prepared in Example 12, as well as native cis-platin (Platinol™, dissolved at 1 mg/ml to produce complete solubility) were infused intravenously into different groups of adult male CBA/J mice. The mice were sacrificed at 15 minutes postinjection, and histologic sections of all organs were stained by an intensified Prussian blue iron reaction, which identified (semiquantitatively) all of the intracellular platinum and most of the extracellular platinum present in target tissues, by staining it aquamarine blue. (This newly developed stain was initially tested on cis-platin in vitro, in order to document the specificity and color of platinum staining.) Results were as follows:
(a) Mice receiving standard intravenous Platinol™ showed moderate-to-intense staining of the liver (centrilobular regions) and almost no staining of lungs.
(b) Mice receiving the reformulated heparin-cisplatin emulsion complex showed moderate-to-intense staining in the lungs, as follows: occasional endothelial staining; substantial staining of lung interstitium, alveolar pneumocytes, respiratory epithelium and bronchial and tracheal lymph nodes; and to a much lesser extent, alveolar macrophages. There was no evidence of pulmonary endothelial toxicity (which, if present, would have appeared as endothelial vesiculation, deposits of extravasated plasma protein, extravasated erythrocytes, and/or intravascular coagulation). Liver staining was approximately 1/10th as abundant as lung staining.

These results establish that high concentrations of Platinol™ (which are usually toxic to endothelium) can be successfully reformulated as a heparin microemulsion complex, and that the heparin component can induce endothelial binding and transcellular uptake of the complexes in a fashion that protects the endothelium from the toxic effects of the drug. These results further indicate that cis-platin (Platinol™) need not be formally microencapsulated in matrix or emulsion carriers, in order to undergo endothelial binding, uptake and preferential tissue access/localization. Rather, it is effective when reformulated on site using a reagent kit (device) comprising heparin.

### EXAMPLE 14

### Epithelial uptake of heparin-iron particles into the lungs following intratracheal administration

Heparin nanospheres, 200-800 nm in diameter, were prepared as in Example 8 (above), except that the metals, iron oxide (Fe₃O₄) and ionic iron (Fe⁺³), were microencapsulated in place of amphotericin B, in order to allow subsequent histochemical identification of the entrapped materials in tissue sections, using the Prussian blue iron stain.

Nanospheres (0.5 cc of a 0.5 mg/ml suspension in 0.1 5M NaCl) were injected into the trachea of pentobarbital-anesthetized adult male CBA/J mice. The mice were sacrificed at 15 minutes postinjection, histologic sections of the lungs prepared, and the sections stained using the standard Prussian blue iron reaction, in order to identify the quantify and positions of microspheres and entrapped iron. The lungs stained positively for microsphere iron. Staining was present in a pattern and intensity identical to those observed following intravenous administration of the amphotericin-containing nanospheres (described in Examples 8 and 9, above). The staining of liver and kidneys was negligible to very low. This established that stabilized heparin nanocarriers (with heparin surfaces) were taken up into lung tissue by epithelial transport, that these carriers deposited a pulmonary reservoir of the entrapped materials (iron compounds), and that a high proportion of the injected dose became localized in the lungs (relative to other organs) when administered via the airways.

### EXAMPLE 15

### Epithelial uptake of heparin nanocarriers into the bladder, small and large bowel (and the draining portal circulation) following intracavitary administration

Nanospheres prepared as in Example 14, were introduced by needle injection into isolated small and large bowel segments, or into the bladder of pentobarbital-anesthetized adult male CBA/J mice. The mice were sacrificed 20 minutes after nanosphere administration, and the tissues prepared and stained as in Example 14. Moderate to marked staining was present in the superficial and deep mucosal layers of both the small and large bowel. Occasional staining was identified in the portal (draining) capillaries and mesenteric lymphatics of the injected bowel segments, and in the deep capillaries of the bladder wall. These results indicated that localized uptake of heparin-coated nanospheres across the bowel and bladder mucosa was achieved by the transepithelial routes. They finally indicated that a portion of the drug particulates taken up via the gastrointestinal tract, were made available for liver and/or systemic distribution via the portal circulation; and that secondary drug targeting at systemic sites is possible following enteric administration of the particulate carriers formulated as in Examples 1-3 and 14.

### EXAMPLE 16

### Preferential accumulation of intravenously administered heparin-coated nanoparticles in the tumors of Buffalo rats bearing Morris 7777-strain hepatocellular carcinomas grown in the liver,lungs and subcutaneous sites

The heparin-iron particulates formulated as in Example 14, were tested, as were heparin-coated, heat-stabilized nanoparticles (200-900 nm in diameter, prepared identically, except using albumin as a matrix; and using beef-lung heparin, Upjohn Co., Kalamazoo, MI, as a surface coating applied by fluid reemulsification). These marker particles were injected intravenously into pentabarbital-anesthetized Buffalo rats bearing 7777-strain Morris hepatocellular carcinomas of the liver or subcutaneous hind limb (primary sites) and the lung (metastatic site). Tumor accumulation and subregion distributions were assessed histochemically (as in Example 14) at intervals of 20 min to 2.5 hrs postinjection. By morphometric analysis, early (20-min) and prolonged (2.5-hr) accumulation was observed in the tumor interstitium, within the tumor cells themselves, and in occasional host macrophages. Such accumulation was observed regardless of the gross anatomic site of tumor. The ratios of stainable tracer metal present in tumor, versus adjacent and distant normal tissues, was between 4:1 and 10:1 (for tumor masses at all three body sites). Importantly, the staining patterns also revealed widespread tumor percolation of the carriers, and further established the preferential accumulation of tracer iron in the following tumor subregions most relevant to drug therapy (diagnosis):
a) at the boundaries between tumor and adjacent normal tissues (i.e., the most actively growing subregions); and
b) at the boundaries between viable and centrally necrotic (dead) tumor, plus the central necrotic regions themselves.

These results indicate that intravenous administration of the heparin-iron nanoparticles allows them to:
(1) distribute via the systemic vasculature,
(2) become selectively localized in a representative malignant tumor,
(3) Percolate widely throughout the tumor interstitium,
(4) become selectively concentrated in the therapeutically and diagnostically relevant subregions of tumor, and
(5) become taken up semiselectively by tumor cells (due to the accentuated cellular uptake of a sulfated polyglucose compound, e.g., heparin, by the induced aniontransport channels of malignant versus normal hepatocyte.

### EXAMPLE 17

### Formulation of heparin-coatable albumin microspheres containing the microencapsulated biomodulator, recombinant human interleukin 2

The biological response modifier, recombinant human interleukin 2 (125-ala-modified IL-2, Amgen Corporation, Thousand Oaks, CA), was microencapsulated in albumin microspheres and nanospheres (with diameters as described in Example 8, above) by emulsion-polymerisation entrapment. In order to maximally preserve biological activity, the albumin-interleukin copolymeric matrix was stabilized by chemical cross-linking with fresh 0.5 to 7.0% formaldehyde (instead of heating), and the cross-linking reaction was quenched with excess glycine. Upon aqueous hydration, IL-2 was released from the spheres in a biologically active form, with a t1/2 of 42 minutes (as assessed by the biological effects of release supernatants on the incorporation of tritiated thymidine by IL-2-dependent T-cell lines tested in vitro). This microsphere formulation of IL-2 was amenable to direct coating (of the preformed particles), both with TDMAC heparin (as described in Example 10, above), and standard heparins (beef-lung heparin, Upjohn Co., Kalamazoo, MI, as described in Example 2.b) and with porcine intestinal mucosal heparin, Sigma Chemicals, St. Louis, MO). These results establish that the biological response modifier, IL-2, which comprises a globular protein, can be entrapped in particulate carriers which become selectively localized in tumors and/or the lungs and brain (as described above).

## Claims

1. A drug carrier or a diagnostic carrier composition comprising a drug or diagnostic agent in combination with a multivalent binding substance which binds to determinants of endothelia or epithelia that separate the blood compartment or external body surfaces from underlying tissues or sites of disease. said binding substance inducing transendothelial or transepithelial transport of the drug or diagnostic agent across said endothelia or epithelia into proximal tissues or sites of disease, said composition comprising a carrier having a non-embolizing size of less than 3 µm.

2. The composition of claim 1 wherein said non-embolizing carrier has a size of less than about 0.2 µm.

3. The composition of clam 1 wherein sad binding substance is selected from carbohydrates. poly-, oligo- or monosaccharides, proteins or peptides, glycosaminoglycans or negatively charged polysaccharides and oligosaccharides.

4. The composition of claims 1 or 2 wherein said binding substance is selected from lisinopril, benzoylphenylalanyl-alanylprolin, intercellular adhesion molecule ICAM-1, chondroitin sulfate, dextran sulfate, cytoadhesion molecules, interleukin-1, endothelial-binding ligands, or, lisinopril conjugates.

5. The composition of any of claims 1 to 3 wherein said binding substance is dermatan sulfate.

6. The composition of any of claims 1 to 3 wherein said binding substance is heparin, a heparin fragment, heparan sulfate, heparin synthetic analogues or heparin sulfate.

7. The composition of claim 1 wherein said carrier comprises one or more of macromolecules, microaggregates, microparticles and emulsions.

8. The composition of clam 1 wherein said drug or diagnostic agent is contained by the carrier.

9. The composition of claim 7 wherein said microparticles are coated with heparin, heparin fragments or synthetic heparin analogues which bind to the complementary heparins and heparan sulfates present on endothelia or epithelia.

10. The composition of claim 1 wherein the drug carrier or diagnostic carrier composition comprises carriers having a matrix containing protamine and a bound surface coating of heparin.

11. The composition of claim 1, wherein said drug carrier or diagnostic carrier composition comprises carriers having a matrix containing a polyamine or polyimine.

12. The composition of claim 1 wherein the drug carrier or diagnostic carrier composition comprises a carrier having a matrix containing either pentosan polysulfate or a poly-COOH compound.

13. The composition of claim 1 wherein said binding substance binds preferentially to activated or deseased endothelium and has the property of inducing active endothelial transport.

14. The composition of claim 1 wherein said binding substance also binds to subendothelial molecules and structures exposed by endothelial activation or disease.

15. The composition of claims 1 wherein said determinants are on or physically associated with endothelia or epithelia, and the binding substance is one or more of interleukin 1, heparan sulfate or an endothelial cytoadhesion molecule.

16. The composition of claim 1 wherein the drug or diagnostic agent is an antifungal drug, in particular amphotericin B.

17. The composition of claim 1 wherein the drug or diagnostic agent is an antimicrobial drug selected from aminoglycosides, cephalosporins or antiviral agents.

18. The composition of claim 1 wherein the drug or diagnostic agent is an antitumor drug, in particular cisplatin.

19. The composition of claim 1 wherein the drug or diagnostic agent is an antiinflammatory agent, in particular steroids, steroid analogs or penicillamine.

20. The composition of claim 1 wherein the drug or diagnostic agent is a cardiovascular or pulmonary drug, in particular heparin, tissue plasminogen activator, probucol or other antilipidemic agent, an anti-asthmatic agent or platelet activating factor inhibitor.

21. The composition of claim 1 wherein the drug or diagnostic agent is a biological response modifer, in particular interleukin 2 (IL-2), tumor necrosis factor (TNF), peptide 11 or other antimetastatic peptide.

22. The composition of claim 1 wherein the drug or diagnostic carrier is a microparticle containing entrapped amphotericin B at a percentage greater than 70 %, solubilized with a polyoxyethylene-polyoxypropylene block copolymer, in a stabilized matrix coated with heparin.

23. The composition of claim 1 wherein the drug or diagnostic carrier is a microparticle containing amphotericin B, solubilized with gamma cyclodextrin, in a stabilized matrix coated with heparin.

24. The composition of claim 1 wherein the drug or diagnostic carrier is a microparticle for which the endothelial or epithelial binding substance is covered by a removable coating which renders the binding substance unexposed to external contact, wherein the removable coating is subject to removal by a triggering event selected from lowered pH, temperature alteration, contact with normal or abnormal endothelia, altered enzyme levels, radiofrequency or ultrasound energy, magnetism, or electricity.

25. The composition of claim 1 wherein the drug or diagnostic agent is bound to carrier substituents to form a paired ion complex.

26. The composition of claim 1 wherein the drug or diagnostic agent is the antitumor drug cisplatin formulated as a paired ion complex, wherein the resulting cisplatin heparin complex is stable in vivo by the criterion of histologically documented extravascular translocation of the combined platinum and heparin components across intact lung-capillary endothelium following intravenous administration.

27. The composition of claim 1 wherein the drug or diagnostic agent is iron oxide or ionic iron.

28. The composition of claim 1 wherein the binding substance is the same as the drug or diagnostic agent, and comprises a microsphere or molecular microaggregate of heparin, heparan sulfate, heparin fragments, synthetic heparin analogs, dermatan sulfate or dextran sulfate.

29. Use of a drug carrier or a diagnostic carrier composition comprising a drug or diagnostic agent in combination with a multivalent binding substance which binds to determinants of endothelia or epithelia that separate the blood compartment or external body surfaces from underlying tissues or sites of disease, said binding substance inducing transendothelial or transepithelial transport of the drug or diagnostic agent across said endothelia or epithelia into proximal tissues or sites of disease, said composition comprising a carrier having a non-embolizing size of less than 3 µm; for the manufacture of a medicament for the treatment or diagnosis of fungal infections, microbial infections, viral infections, inflammation, abscesses, tumors, cardiovascular abnormalities, pulmonary abnormalities, multiple sclerosis, thrombosis, infarcts, diabetic angiopathy, acute and chronic arthritis, disseminated intravascular coagulation, genetic diseases, degenerative diseases, endometriosis, infertility, allograft rejection, asthma, pulmonary emphysema, foci of tissue or endothelial disease.

30. Use as claimed in claim 29 wherein transcellular transport is completed within twelve minutes of endothelial/epithelial contact under *in vivo* conditions of microvascular blood flow.

31. Use as claimed in claim 29 or 30 wherein said binding substance is selected from heparin, a heparin fragment, heparin synthetic analogues, heparin sulfate or heparan sulfate.

32. Use as claimed in claims 29 or 30 wherein said binding substance is dermatan sulfate.

33. Use as claimed in any of claims 29 to 32 wherein the drug carrier is in a pharmaceutically acceptable solution suitable for intravascular or other parenteral injection.

## Patentansprüche

1. Trägerzusammensetzung für ein Arzneimittel oder diagnostisches Mittel, die ein Arzneimittel oder diagnostisches Mittel in Kombination mit einer mehrwertigen Bindungssubstanz umfaßt, welche an Endothel- oder Epithel-Determinanten bindet, die die Blutkompartimente oder die äußeren Körperoberflächen von darunter liegenden Geweben oder Krankheitsherden trennen, wobei die Bindungssubstanz den transendothelialen oder transepithelialen Transport des Arzneimittels oder des diagnostischen Mittels durch die Endothelien oder Epithelien in sich anschließende Gewebe oder Krankheitsherde induziert, wobei die Zusammensetzung einen Träger mit einer nicht-embolisierenden Größe von weniger als 3 µm umfaßt.

2. Zusammensetzung nach Anspruch 1, wobei der nicht-embolisierende Träger eine Größe von weniger als etwa 0,2 µm hat.

3. Zusammensetzung nach Anspruch 1, wobei die Bindungssubstanz ausgewählt ist aus Kohlenhydraten, Poly-, Oligo- oder Monosacchariden, Proteinen oder Peptiden, Glykosaminglykanen oder negativ geladenen Polysacchariden und Oligosacchariden.

4. Zusammensetzung nach Anspruch 1 oder 2, wobei die Bindungssubstanz ausgewählt ist aus Lisinopril, Benzoyl-Phenylalanyl-Alanylprolin, dem intrazellulären Adhäsionsmolekül ICAM-1, Chondroitinsulfat, Dextransulfat, Cytoadhäsionsmolekülen, Interleukin-1, endothelialen Bindungsliganden oder Lisinoprilkonjugaten.

5. Zusammensetzung nach einem der Ansprüche 1 bis 3, wobei die Bindungssubstanz Dermatansulfat ist.

6. Zusammensetzung nach einem der Ansprüche 1 bis 3, wobei die Bindungssubstanz Heparin, ein Heparinfragment, Heparansulfat, synthetische Heparinanaloga oder Heparinsulfat ist.

7. Zusammensetzung nach Anspruch 1, wobei der Träger ein oder mehrere makromolekülen, Mikroaggregaten, Mikropartikeln und Emulsionen umfaßt.

8. Zusammensetzung nach Anspruch 1, wobei das Arzneimittel oder diagnostische Mittel in dem Träger enthalten ist.

9. Zusammensetzung nach Anspruch 7, wobei die Miloropartikel mit Heparin, Heparinfragmenten oder synthetischen Heparinanalogen beschichtet sind, welche an die komplementären Heparine und Heparansulfate, die auf den Endothelien oder Epithelien anwesend sind, binden.

10. Zusammensetzung nach Anspruch 1, wobei die Trägerzusammensetzung für ein Arzneimittel oder diagnostisches Mittel Träger mit einer Matrix umfaßt, die Protamin und eine gebundene Oberflächenbeschichtung von Heparin enthält.

11. Zusammensetzung nach Anspruch 1, wobei die Trägerzusammensetzung für ein Arzneimittel oder diagnostisches Mittel Träger mit einer Matrix umfaßt, die ein Polyamin oder Polyimin enthält.

12. Zusammensetzung nach Anspruch 1, wobei die Trägerzusammensetzung für ein Arzneimittel oder diagnostisches Mittel einen Träger mit einer Matrix umfaßt, die entweder Pentosanpolysulfat oder eine Poly-COOH-Verbindung enthält.

13. Zusammensetzung nach Anspruch 1, wobei die Bindungssubstanz vorzugsweise an aktiviertes oder erkranktes Endothelium bindet und die Eigenschaft hat, den aktiven endothelialen Transport zu induzieren.

14. Zusammensetzung nach Anspruch 1, wobei sich die Bindungssubstanz auch an subendotheliale Moleküle und Strukturen bindet, die durch endotheliale Aktivierung oder Erkrankung exponiert worden sind.

15. Zusammensetzung nach Anspruch 1, wobei sich die Determinanten auf Endothelien oder Epithelien befinden oder körperlich mit Endothelien oder Epithelien assoziiert sind, und die Bindungssubstanz eine oder mehrere von Interleukin 1, Heparansulfat oder ein endotheliales Zelladhäsionsmolekül ist.

16. Zusammensetzung nach Anspruch 1, wobei das Arzneimittel oder diagnostische Mittel ein Anti-Pilz-Arzneimittel, insbesondere Amphotericin B, ist.

17. Zusammensetzung nach Anspruch 1, wobei das Arzneimittel oder diagnostische Mittel ein anti-mikrobiales Arzneimittel ausgewählt aus Aminoglykosiden, Cephalosporinen oder antiviralen Mitteln ist.

18. Zusammensetzung nach Anspruch 1, wobei das Arzneimittel oder diagnostische Mittel ein Anti-Tumor-Arzneimittel, insbesondere cis-Platin, ist.

19. Zusammensetzung nach Anspruch 1, wobei das Arzneimittel oder diagnostische Mittel ein Anti-Entzündungs-Arzneimittel ist, insbesondere Steroide, Steroidanaloge oder Penicillamin.

20. Zusammensetzung nach Anspruch 1, wobei das Arzneimittel oder diagnostische Mittel ein kardiovaskulares oder Lungen-Arzneimittel ist, insbesondere Heparin, Gewebeplasminogenaktivator, Probukol oder ein anderes antilipidemisches Mittel, ein anti-asthmatisches Mittel oder plättchen-aktivierender Faktor-Inhibitor (platelet activating factor inhibitor).

21. Zusammensetzung nach Anspruch 1, wobei das Arzneimittel oder diagnostische Mittel ein Modifikationsmittel der biologischen Antwort ist, insbesondere Interleukin 2 (IL-2), Tumornekrosefaktor (TNF), Peptid 11 oder ein anderes anti-metastatisches Peptid.

22. Zusammensetzung nach Anspruch 1, wobei der Träger für ein Arzneimittel oder diagnostische Mittel ein Mikropartikel ist, das eingeschlossenes Amphotericin B zu einem Prozentsatz größer als 70% enthält, solubilisiert mit einem Polyoxyethylen-Polyoxypropylen-Block-copolymer, in einer stabilisierten mit Heparin beschichteten Matrix.

23. Zusammensetzung nach Anspruch 1, wobei der Träger für ein Arzneimittel oder diagnostische Mittel ein Mikropartikel ist, das Amphotericin B enthält, solubilisiert mit Gammacyclodextrin, in einer stabilisierten mit Heparin beschichteten Matrix.

24. Zusammensetzung nach Anspruch 1, wobei der Träger für ein Arzneimittel oder diagnostische Mittel ein Mikropartikel ist, für den die endotheliale oder epitheliale Bindungssubstanz mit einer entfernbaren Beschichtung bedeckt ist, die die Bindungssubstanz von einem äußeren Kontakt abhält, wobei die entfernbare Beschichtung einer Entfernung durch ein auslösendes (triggerndes) Ereignis ausgewählt aus erniedrigtem pH, Temperaturveränderung, Kontakt mit normalen oder abnormalen Endothelien, veränderten Enzym-Level, Hochfrequenz- oder Ultraschallenergie, Magnetismus oder Elektrizität zugänglich ist.

25. Zusammensetzung nach Anspruch 1, wobei das Arzneimittel oder diagnostische Mittel an Trägersubstituenten gebunden ist, um einen gepaarten Ionenkomplex zu bilden.

26. Zusammensetzung nach Anspruch 1, wobei das Arzneimittel oder diagnostische Mittel das Anti-Tumor-Arzneimittel cis-Platin, formuliert als ein gepaarter Ionenkomplex ist, wobei der sich ergebende cis-Platin-Heparin-Komplex in vivo stabil ist, und zwar nach dem Kriterium histologisch dokumentierter extravaskularer Translokation der kombinierten Platin- und Heparin-Komponenten über intaktes Lungenkapillar-Endothelium, im Anschluß auf intravenöse Verabreichung.

27. Zusammensetzung nach Anspruch 1, wobei das Arzneimittel oder diagnostische Mittel Eisenoxid oder ionisches Eisen ist.

28. Zusammensetzung nach Anspruch 1, wobei die Bindungssubstanz die gleiche wie das Arzneimittel oder diagnostische Mittel ist und eine Mikrosphäre oder ein molekulares Mikroaggregat von Heparin, Heparansulfat, Heparinfragmenten, synthetischen Heparinanalogen, Dermatansulfat oder Dextransulfat enthält.

29. Verwendung einer Trägerzusammensetzung für ein Arzneimittel oder diagnostisches Mittel, die ein Arzneimittel oder diagnostisches Mittel in Kombination mit einer mehrwertigen Bindungssubstanz umfaßt, welche an Endothel- oder Epithel-Determinanten bindet, die die Blutkompartimente oder die äußeren Körperoberflächen von darunter liegenden Geweben oder Krankheitsherden trennen, wobei die Bindungssubstanz den transendothelialen oder transepithelialen Transport des Arzneimittels oder des diagnostischen Mittels durch die Endothelien oder Epithelien in sich anschließende Gewebe oder Krankheitsherde induziert, wobei die Zusammensetzung einen Träger mit einer nicht-embolisierenden Größe von weniger als 3 µm umfaßt; für die Herstellung eines Medikaments für die Behandlung oder Diagnose von Pilzinfektionen, mikrobialen Infektionen, viralen Infektionen, Entzündung, Abszessen, Tumoren, kardiovaskulären Anomalien, Lungenanomalien, Multipler Sklerose, Thrombose, Infarkten, diabetischer Angiopathie, akuter und chronischer Artritis, disseminierter intravaskulärer Koagulation, genetischer Erkrankungen, degenerativen Erkrankungen, Endometrioses, Infertilität, Allotransplantat-Abstoßung, Asthma, Lungenemphysem, Herden von Gewebe- oder endothelialer Erkrankung.

30. Verwendung nach Anspruch 29, wobei der transzelluläre Transport innerhalb von 12 Minuten des Endothelial/Epithelial-Kontaktes unter *in vivo*-Bedingungen von mikrovaskulärem Blutfluß vollendet wird.

31. Verwendung nach Anspruch 29 oder 30, wobei die Bindungssubstanz ausgewählt ist aus Heparin, einem Heparinfragment, synthetischem Heparinanaloga, Heparinsulfat oder Heparansulfat.

32. Verwendung nach Anspruch 29 oder 30, wobei die Bindungssubstanz Dermatansulfat ist.

33. Verwendung nach einem der Ansprüche 29-32, wobei der Arzneimittelträger in einer pharmazeutisch verträglichen Lösung vorliegt, die für die intravaskuläre oder andere parenterale Injektion geeignet ist.

## Revendications

1. Une composition de véhicule de médicament ou de véhicule de diagnostic, comprenant un agent médicamenteux ou de diagnostic en combinaison avec une substance liante multivalente se liant à des déterminants d'endothéliums ou d'épithéliums séparant le compartiment sanguin ou des surfaces externes du corps d'avec des tissus sous-jacents ou des sites de maladie, cette substance liante induisant un transport transendothélial ou transépithélial de l'agent médicamenteux ou de diagnostic au travers des endothéliums ou des épithéliums jusque dans des sites de maladie ou des tissus proximaux, cette composition comprenant un véhicule possédant une dimension non emboligènes inférieur à 3 µm.

2. La composition de la revendication 1, dans laquelle ce véhicule non emboligène a une dimension inférieur à 0.2 µg.

3. La composition de la revendication 1, dans laquelle la substance liante est choisie parmi les hydrates de carbone, les polysaccharides, oligosaccharides ou monosaccharides, les protéines ou les peptides, les glycosaminoglycanes ou les polysaccharides et oligosaccharides négativement chargés.

4. La composition de la revendication 1 ou 2, dans laquelle la substance liante est choisie parmi le lisinopril, la benzoyl-phénylalanylalanylproline, la molécule d'adhésion intercellulaire ICAM-1, le sulfate de chondroïtine, le sulfate de dextrane, des molécules de cytoadhésion, l'interleukine-1, des ligands de liaison endothéliale, ou les conjugués du lisinopril.

5. La composition selon une des revendications 1 à 3, dans laquelle la substance liante est le sulfate de dermatane.

6. La composition selon une des revendications 1 à 3, dans laquelle la substance liante est l'héparane, un fragment d'héparine, le sulfate d'héparane, les analogues synthétiques de l'héparine ou le sulfate d'héparine.

7. La composition de la revendication 1, dans laquelle ledit véhicule comprend l'une ou plusieurs des formes du groups des macromolécules, microagrégats, micorparticules et émulsions.

8. La composition de la revendication 1, dans laquelle l'agent médicamenteux ou de diagnostic est contenu par le véhicule.

9. La composition de la revendication 7, dans laquelle lesdites microparticules sont revêtues d'héparine, de fragments d'héparine ou d'analogues de synthèse de l'héparine qui se lient aux héparines complémentaires et aux sulfates d'héparane présents sur les endothéliums ou les épithéliums.

10. La composition de la revendication 1, dans laquelle la composition de véhicule de médicament ou de véhicule de diagnostic comprend des véhicules possédant une matrice contenant de la protamine et un revêtement de surface liée d'héparine.

11. La composition de la revendication 1, dans laquelle la composition de véhicule de médicament ou de véhicule de diagnostic comprend des véhicules possédant une matrice contenant un polyamine ou un polyimine.

12. La composition de la revendication 1, dans laquelle la composition de véhicule de médicament ou de véhicule de diagnostic comprend un véhicule possédant une matrice contenant soit du polysulfate de pentosane soit un composé poly-COOH.

13. La composition de la revendication 1, dans laquelle la substance liante se lie préférentiellement à un endothélium activé ou atteint de maladie et possède la propriété d'induire un transport endothélial actif.

14. La composition de la revendication 1, dans laquelle la substance liante se lie aussi à des molécules et structures subendothéliales exposées par la maladie ou l'activation endothéliale.

15. La composition de la revendication 1, dans laquelle lesdits déterminants sont sur les endothéliums ou les épithéliums, ou physiquement associés à ceux-ci, et la substance liante est l'une ou plusieurs des substances du groupe de l'interleukine-1, du sulfate d'héparane ou d'une molécule de cytoadhésion endothéliale.

16. La composition de la revendication 1, dans laquelle l'agent médicamenteux ou de diagnostic est un médicament antifongique, en particulier l'amphotéricine B.

17. La composition de la revendication 1, dans laquelle l'agent médicamenteux ou de diagnostic est un agent antimicrobien choisi parmi les aminoglycosides, les céphalosporines ou les agents antiviraux.

18. La composition de la revendication 1, dans laquelle l'agent médicamenteux ou de diagnostic est un médicament antitumoral, en particulier la cisplatine.

19. La composition de la revendication 1, dans laquelle l'agent médicamenteux ou de diagnostic est un agent anti-inflammatoire, en particulier des stéroïdes, des analogues de stéroïdes ou la pénicillamine.

20. La composition de la revendication 1, dans laquelle l'agent médicamenteux ou de diagnostic est un médicament cardio-vasculaire ou pulmonaire, en particulier l'héparine, l'activateur du plasminogène tissulaire, le probucol ou autre agent antilipidémique, un agent antiasthmatique ou un inhibiteur du facteur d'activation plaquettaire.

21. La composition de la revendication 1, dans laquelle l'agent médicamenteux ou de diagnostic est un modificateur de réponse biologique, en particulier l'interleukine-2 (IL-2), le facteur de nécrose tumorale (TNF), le peptide 11 ou un autre peptide antimétastatique.

22. La composition de la revendication 1, dans laquelle le véhicule de médicament ou de diagnostic est une microparticule contenant de l'amphotéricine B piégée en proportion supérieure à 70 %, solubilisé par un copolymère bloc polyoxyéthlyène-polyoxypropylène, dans une matrice stabilisée revêtue d'héparine.

23. La composition de la revendication 1, dans laquelle le véhicule de médicament ou de diagnostic est une microparticule contenant de l'amphotéricine B, solubilisée par la gamma-cyclodextrine, dans une matrice stabilisée revêtue d'héparine.

24. La composition de la revendication 1, dans laquelle le véhicule de médicament ou de diagnostic est une microparticule pour laquelle la substance liante endothéliale ou épithéliale est recouverte par un revêtement amovible mettant la substance liante à l'abri du contact externe, ce revêtement amovible étant retiré par un événement déclenchant choisi dans le groupe comprenant l'abaissement du pH, la modification de la température, le contact avec des endothéliums normaux ou anormaux, des niveaux enzymatiques modifiés, une énergie radiofréquence ou ultrasonore, le magnétisme ou l'électricité.

25. La composition de la revendication 1, dans laquelle le médicament ou agent de diagnostic est lié à des substituants du véhicule pour former un complexe ionique apparié.

26. La composition de la revendication 1, dans laquelle le médicament ou agent de diagnostic est le médicament antitumoral constitué par la cisplatine formulée en un complexe ionique apparié, le complexe cisplatine-héparine résultant étant stable *in vivo* au regard du critère de la translocation extravasculaire histologiquement documentée des constituants combinés platine et héparine au travers d'un endothélium intact de capillaires pulmonaires après administration intraveineuse.

27. La composition de la revendication 1, dans laquelle le médicament ou agent de diagnostic est l'oxyde de fer ou le fer ionique.

28. La composition de la revendication 1, dans laquelle la substance liante est la même que le médicament ou agent de diagnostic, et comprend une microsphère ou un microagrégat moléculaire d'héparine, de sulfate d'héparane, de fragments d'héparine, d'analogues de synthèse de l'héparine, de sulfate de dermatane ou de sulfate de dextrane.

29. Utilisation d'une composition de véhicule de médicament ou de véhicule de diagnostic, comprenant un agent médicamenteux ou de diagnostic en combinaison avec une substance liante multivalente se liant à des déterminants d'endothéliums ou d'épithéliums séparant le compartiment sanguin ou des surfaces externes du corps d'avec des tissus sous-jacents ou des sites de maladie, cette substance liante induisant un transport transendothélial ou transépithélial de l'agent médicamenteux ou de diagnostic au travers des endothéliums ou des épithéliums jusque dans des sites de maladie ou des tissus proximaux, cette composition comprenant un véhicule possédant une dimension non emboligènes inférieur à 3 µm ; pour la réalisation d'un médicament pour le traitement ou le diagnostic des infections fongiques, microbiques et viraux, inflammation, abcès, tumeurs, anomalies cardiovasculaires et pulmonaires, scléroses multiples, thrombose, infarcts, angiopathie diabétique, arthrose aiguë et chronique, coagulation intravasculair disséminée, maladies génétiques et dégénératives, endométrioses, infertilité, rejet de l'allogreffe, asthmes, emphysèmes pulmonaires, foyers de maladies tissulaires ou endothéliales.

30. L'utilisation de la revendication 29, dans laquelle le transport transcellulaire est accompli dans un délai de 12 minutes de contact endothélial/épithélial dans des conditions *in vivo* de débit sanguin microvasculaire.

31. L'utilisation de la revendication 29 ou 30, dans laquelle cette substance liante est choisie parmi l'héparine, un fragment d'héparine, les analogues synthétiques de l'héparine, le sulfate d'héparine ou le sulfate d'héparane.

32. L'utilisation de la revendication 29 ou 30, dans laquelle la substance liante est le sulfate de dermatane.

33. L'utilisation selon une des revendications 29 ou 32, dans laquelle le véhicule de médicament est dans une solution acceptable de manière pharmaceutique et souhaitable pour une injection intravasculaire ou par d'autres injections parentérales.
